# EUROPEAN PATENT SPECIFICATION

(11) **EP 4 003 470 B1**
(45) Date of publication and mention of the grant of the patent: **05.11.2025**
(21) Application number: 20757175.3
(22) Date of filing: 31.07.2020
(51) Int. Cl.: A61M 15/00, A61B 5/00, G16H 20/00

(54) **MODULAR INHALER ADHERENCE MONITOR**
ADHÄRENZMONITOR FÜR MODULAREN INHALATOR
MONITEUR D'ADHÉRENCE D'INHALATEUR MODULAIRE

(30) Priority: 31.07.2019 US 201962881100 P
(43) Date of publication of application: 01.06.2022
(73) Proprietor: Reciprocal Labs Corporation, Madison, WI 53703 (US)
(72) Inventor: MARKEY, Amber, Michelle, Madison, Wisconsin 53703 (US); KOBLENSKI, Samuel, A., Madison, Wisconsin 53703 (US); BADDELEY, Robert, Louis, Madison, Wisconsin 53703 (US); TRACY, Gregory, Frederick, Madison, Wisconsin 53703 (US)
(74) Representative: Mathys & Squire
(86) International application number: PCT/US2020/044419
(87) International publication number: WO 2021/022118

(56) References cited:
- WO-A1-2015/109259
- WO-A1-2016/033421
- WO-A1-2017/051389
- WO-A1-2017/141194
- WO-A1-2019/012558
- WO-A1-2019/022620
- WO-A1-2020/047102
- US-A1- 2002 000 225
- US-A1- 2014 182 584
- US-A1- 2015 061 867
- US-A1- 2016 144 141
- US-A1- 2018 140 786

## Description

### TECHNICAL FIELD

The present disclosure relates generally to accessories for inhalers, and more specifically for a modular inhaler adherence monitoring device that monitors adherence of an inhaler user. The invention is set out in the appended set of claims.

### BACKGROUND

Currently, many patients with ailments are provided an inhaler to provide dosages of drugs. For example, an asthma patient may be provided a stimulant to assist mucus and reducing inflammation in clearing up breathing passages. Thus, when the patient experiences asthma exacerbations or as a daily maintenance medication to control symptoms, the patient can put the inhaler in front of their mouth and activate the inhaler spray, delivering a dose of the drug into the lungs in order to relieve the symptom.

A known pressurized metered dose inhaler (pMDI) inhaler, such as the AstraZeneca Symbicort Rapihaler^{®}, for inhalation of drugs has an actuator housing at least partially defining a flow passageway extending through the inhaler from an air inlet to an outlet. A pressurized metered dose canister is held by the actuator. The canister includes a valve stem and a metering valve arranged to seat in a valve stem block formed on the housing and a main canister body of the canister may be moved relative to the housing and valve stem so as to operate the metering valve and fire a metered dose of propellant and active drug through the valve stem block and into the flow passageway. By depressing the canister, when a user inhales through a mouthpiece of the housing, air may be drawn into the housing between the canister and an inner wall of the housing, and may flow along past the canister towards the outlet. The Symbicort Rapihaler^{®} delivers a combination of budesonide and formoterol (ICS/LABA combination) to treat asthma and/or chronic obstructive pulmonary disease (COPD). Other types of inhalers may deliver other kinds of medicaments for such ailments and other ailments. Such inhalers include the Orion Easyhaler^{®} and the Teva Redihaler^{®}.

One significant problem is users who often do not properly operate the inhaler, thus resulting in ineffective delivery of the medicament. For example, a user may not inhale when the medicament is dispensed from the canister or hold the mouthpiece in proper relation to the mouth. Unfortunately, there is no effective method to determine whether a user is following a correct technique other than health care professionals reviewing long term health improvement after use of an inhaler for a period of time.

The use of an adherence monitor integrated in the inhaler itself has been proposed, but such a solution is expensive and as the inhalers are designed to be disposable, such a solution is not practical. Another solution is to have an attachable monitor that may be attached to existing inhalers such as the Symbicort Rapihaler^{®}. However, the unique shape of the Symbicort Rapihaler^{®} and other similar inhalers make the physical design of such a monitor challenging. Specifically, there is a significant amount of flex and wiggle of the canister relative to the inhaler body. The amount of flexibility exceeds the amount of change when the canister was pressed, making it impossible to use a limit switch to detect actuation of the inhaler.

Further, the canister of the Symbicort Rapihaler^{®} has a label and a dose counter on the front and top that cannot be covered by the body of an adherence monitor. These features make it difficult to attach to anything but the body, and sense only on the edge of the front of the canister, which had the least amount of play relative to the inhaler body. The mouthpiece cover covers a substantial amount of the inhaler, and must be kept exposed. Further, the strap on the bottom of the inhaler that holds the mouthpiece cover must also be exposed. This further limits the availability of the attachment.

Examples of the prior art for inhalers with adherence-monitoring functionality are WO 2017/141194 A1, WO 2019/012558 A1, US 2016/144141 A1, WO 2015/109259 A1, US 2014/182584 A1, WO 2017/051389 A1, WO 2016/033421 A1 and WO 2019/022620 A1.

There is a need for a modular adherence monitor that may be attached to an existing inhaler to collect data related to adherence of use of the existing inhaler. There is another need for an adherence monitor that has multiple sensors to determine the actuation of an attached inhaler and an inhalation event. There is another need for a modular adherence sensor that does not interfere with or modify the use of an inhaler. There is another need for a modular adherence sensor that applies a time stamp and inhalation data to an actuation event of the inhaler.

### SUMMARY

The invention is set out in the appended claims.

One disclosed example is an adherence monitor for attachment to an inhaler. The inhaler has a drug canister covered by a canister cover, an actuator holding the drug canister, and a dosing device operable to allow the drug canister to be actuated to release a dose. The actuator has a mouthpiece. The monitor includes an actuation detection sensor operable to sense the physical movement of the drug canister when actuated. The monitor includes an inhalation data sensor operable to sense air pressure change created by inhalation of the dose from the actuation. A controller is coupled to the actuation detection sensor and the inhalation data sensor to record an actuation event.

A further implementation of the example adherence monitor is an embodiment including an accelerometer coupled to the controller. The accelerometer outputs a signal indicative of movement of the inhaler prior to actuation. The controller activates the sensors when the movement is detected. Another implementation is where the actuation detection sensor is an infrared sensor. Another implementation is where the actuation detection sensor is a contact switch. Another implementation is where the actuation detection sensor is a barometric pressure sensor. Another implementation is where the inhaler includes a shield attached to the canister cover. The actuation detection sensor detects the movement of the shield as indicating actuation of the inhaler. Another implementation is where the inhalation data sensor is a pressure sensor. The controller determines a pressure curve during inhalation of the dose from the inhaler. Another implementation is where the controller applies a time stamp to collected data indicating actuation of the inhaler. Another implementation is where the adherence monitor includes a transceiver coupled to the controller. The controller sends the data based on the actuation event to an external client device in communication with the transceiver. Another implementation is where the external device is a mobile computing device associated with the user. The external device executes an application to analyze the collected data to determine adherence. Another implementation is where the adherence monitor includes an attachment detection sensor to detect when the inhaler is attached to the adherence monitor. Another implementation is where the adherence monitor includes an activation button operable by a user to activate the controller and the sensors. Another implementation is where the adherence monitor includes a body fitting over the canister cover of the inhaler. Another implementation is where the inhalation data sensor is positioned on a circuit board to be exposed to a gap between the adherence monitor and the canister cover of the inhaler.

Another example is an adherence monitor for attachment to an inhaler. The inhaler has a drug canister, an actuator holding the drug canister, the actuator having a cylindrical body having one end holding the drug canister with a mouthpiece on the opposite end, and a dosing device attached to the drug canister. The dosing device includes a front shield surface and is operable to allow the drug canister to be actuated to release a dose. The monitor includes a pair of curved side walls that conform to the sides of the cylindrical body of the actuator. Each of the side walls has an open front edge and a closed back edge. A side arm is attached to one of the side walls to overlap the cylindrical body of the actuator. The mouthpiece of the inhaler is accessible and the front shield surface is exposed. An electronics housing is attached to the closed back edges of the side walls.

A further implementation of the example adherence monitor is an embodiment including an actuation detection sensor operable to sense the physical movement of the drug canister when actuated. An inhalation data detection sensor senses air pressure change created by the actuation. A controller in the electronics housing is coupled to the sensors to record an actuation event. Another implementation is where the inhaler includes a strap connecting a cover to the actuator. The side walls each include a bottom section that form a slot for the strap. Another implementation is where the adherence monitor includes a printed circuit board in the electronics housing. The printed circuit board has a first surface including a connector connected to the actuation detection sensor. The actuation detection sensor is mounted on one of the side walls in proximity to the shield surface of the inhaler. The circuit board includes a second opposite surface mounting the inhalation data sensor in proximity to a gap between a top cover of the side walls and the actuator of the inhaler. Another implementation is where the adherence monitor includes an attachment detection sensor mounted on the first surface of the printed circuit board operable to detect the attachment of the inhaler to the adherence monitor. Another implementation is where the electronics housing includes a back panel having an activation button to activate the controller and the sensors.

Another example is an adherence monitor for attachment to an inhaler. The inhaler has a drug canister, an actuator holding the drug canister, and a canister cover covering the drug canister. The actuator has one end holding the drug canister with a mouthpiece on the opposite end. The drug canister may be actuated to release a dose. The monitor includes a body fitting around the canister cover. An electronics housing is attached to the body. An actuation detection sensor is operable to sense the actuation of the drug canister. An inhalation data detection sensor is operable to sense air pressure change created by the actuation. A controller in the electronics housing id coupled to the sensors to record an actuation event.

A further implementation of the example adherence monitor is an embodiment including a printed circuit board in the electronics housing. The printed circuit board has a first surface with the actuation detection sensor. The printed circuit board has a second opposite surface mounting the inhalation data sensor in proximity to a gap between a top cover of the electronics housing and the canister cover of the inhaler. Another implementation is where the electronics housing includes a panel having an activation button operable to activate the controller and the sensors. Another implementation is where the canister cover is physically moveable to actuate the drug canister. Another implementation is where the actuation detection sensor is a limit switch activated when the canister cover is moved. Another implementation is where the actuation detection sensor and the inhalation detection sensor are a barometric pressure sensor.

The above summary is not intended to represent each embodiment or every aspect of the present disclosure. Rather, the foregoing summary merely provides an example of some of the novel aspects and features set forth herein. The above features and advantages, and other features and advantages of the present disclosure, will be readily apparent from the following detailed description of representative embodiments and modes for carrying out the present technology.

### BRIEF DESCRIPTION OF THE DRAWINGS

The disclosure will be better understood from the following description of exemplary embodiments together with reference to the accompanying drawings, in which:
FIG. 1A is a perspective view of a commercially available prior art inhaler;
FIG. 1B is an exemplary perspective view of the inhaler in FIG. 1A, which is attached with an adherence monitor in accordance with the present invention;
FIG. 2A is a perspective view of the inventive adherence monitor attached to the inhaler shown in FIG. 1A;
FIG. 2B is a back perspective view of the inventive adherence monitor attached to the inhaler shown in FIG. 1A;
FIG. 2C is a top perspective view of the inventive adherence monitor attached to the inhaler shown in FIG. 1A;
FIG. 2D is a bottom perspective view of the inventive adherence monitor attached to the inhaler shown in FIG. 1A;
FIG. 2E is a side view of the inventive adherence monitor attached to the inhaler shown in FIG. 1A;
FIG. 2F is an opposite side view of the inventive adherence monitor attached to the inhaler shown in FIG. 1A;
FIG. 2G is front view of the inventive adherence monitor attached to the inhaler shown in FIG. 1A;
FIG. 2H is a back view of the inventive adherence monitor attached to the inhaler shown in FIG. 1A;
FIG. 2I is a top view of the inventive adherence monitor attached to the inhaler shown in FIG. 1A;
FIG. 2J is a bottom view of the inventive adherence monitor attached to the inhaler shown in FIG. 1A;
FIG. 3A is a perspective back cutaway view of the inventive adherence monitor in FIG. 1B showing internal electronic components mounted on a circuit board;
FIG. 3B is a perspective front cutaway view of the inventive adherence monitor in FIG. 1B showing the rear surface of the circuit board;
FIG. 4 is a cutaway view of the inventive adherence monitor and the inhaler in FIG. 1B during operation of the inhaler;
FIG. 5 is a cutaway side view of the rear surface of the circuit board of the inventive adherence monitor in FIG. 1B;
FIG. 6 is a circuit diagram of the electronic components of the inventive adherence monitor in FIG. 1B;
FIG. 7 is a state diagram of the routine that operates the inventive adherence monitor in FIG. 1B;
FIG. 8 is a flow diagram of the routine that operates the inventive adherence monitor in FIG. 1B;
FIG. 9A is a perspective view of another example of a known prior art inhaler;
FIG. 9B is a front perspective view of an example type of modular adherence monitor attached to the inhaler shown in FIG. 9A;
FIG. 9C is a rear perspective view of the modular adherence monitor attached to the inhaler shown in FIG. 9A;
FIG. 9D is a side view of the modular adherence monitor attached to the inhaler shown in FIG. 9A;
FIG. 9E is an opposite side view of the modular adherence monitor attached to the inhaler shown in FIG. 9A;
FIG. 9F is a front view of the modular adherence monitor attached to the inhaler shown in FIG. 9A;
FIG. 9G is a top view of the modular adherence monitor attached to the inhaler shown in FIG. 9A;
FIG. 9H is a rear view of the modular adherence monitor attached to the inhaler shown in FIG. 9A;
FIG. 10A is a cutaway view showing a printed circuit board of the modular adherence monitor shown in FIG. 9B
FIG. 10B is a front view of the printed circuit board in FIG. 10A;
FIG. 10C is a rear view of the printed circuit board in FIG. 10A;
FIG. 11A is a cutaway view showing the adherence monitor and the inhaler in FIG. 9B during operation of the inhaler;
FIG. 11B is a cutaway side view of the rear surface of the circuit board of the adherence monitor in FIG. 9B;
FIG. 12A is a state diagram of the event detection routine of the adherence monitor in FIG. 9B;
FIG. 12B is a state diagram of the shake detection routine of the adherence monitor in FIG. 9B;
FIG. 13A is a perspective view of another example of a known prior art inhaler;
FIG. 13B is a front perspective view of another example type of modular adherence monitor attached to the inhaler shown in FIG. 13A;
FIG. 13C is a rear perspective view of the modular adherence monitor attached to the inhaler shown in FIG. 13A;
FIG. 13D is a side view of the modular adherence monitor attached to the inhaler shown in FIG. 13A;
FIG. 13E is an opposite side view of the modular adherence monitor attached to the inhaler shown in FIG. 13A;
FIG. 13F is a front view of the modular adherence monitor attached to the inhaler shown in FIG. 13A;
FIG. 13G is a top view of the modular adherence monitor attached to the inhaler shown in FIG. 13A;
FIG. 13H is a rear view of the modular adherence monitor attached to the inhaler shown in FIG. 13A;
FIG. 14A is a front view of the circuit board in the adherence monitor in FIG. 9B;
FIG. 14B is a rear view of the circuit board in the adherence monitor in FIG. 9B;
FIG. 15A is a cutaway rear view of the adherence monitor and the inhaler in FIG. 13B during operation of the inhaler;
FIG. 15B is a cutaway side view of the rear surface of the circuit board of the adherence monitor in FIG. 13B;
FIG. 15C is a cutaway front side view of the vents of the inhaler in relation to the adherence monitor in FIG. 13B;
FIG. 16 is a state diagram of the routine that operates the adherence monitor in FIG. 13B; and
FIG. 17 is a block diagram of a health care system that supports the data obtained by the example adherence monitors.

The present disclosure is susceptible to various modifications and alternative forms. Some representative embodiments have been shown by way of example in the drawings and will be described in detail herein.

### DETAILED DESCRIPTION OF THE ILLUSTRATED EMBODIMENTS

The present technology can be embodied in many different forms. Representative embodiments are shown in the drawings, and will herein be described in detail. The present disclosure is an example or illustration of the principles of the present disclosure. ,For purposes of the present detailed description, unless specifically disclaimed, the singular includes the plural and vice versa; and the word "including" means "including without limitation." Moreover, words of approximation, such as "about," "almost," "substantially," "approximately," and the like, can be used herein to mean "at," "near," or "nearly at," or "within 3-5% of," or "within acceptable manufacturing tolerances," or any logical combination thereof, for example.

The present disclosure relates to a modular adherence attachment intended to monitor use of an inhaler. One example is a modular adherence attachment intended to monitor use of the AstraZeneca Symbicort^{®} pressurized metered dose inhaler (pMDI) form factor. The adherence monitor includes physical features that wrap around the inhaler and thus attach the adherence monitor to the AstraZeneca Symbicort^{®} inhaler.

Another example modular adherence attachment is intended to monitor use of the Orion Easyhaler^{®} pressurized metered dose inhaler (pMDI) form factor. The adherence monitor includes physical features that mate with the actuator of the inhaler and thus attach the adherence monitor to the Orion Easyhaler^{®} inhaler to detect actuation of the inhaler.

Another example modular adherence attachment intended to monitor use of the Teva Redihaler^{®} pressurized metered dose inhaler (pMDI) form factor. The adherence monitor includes physical features that mate with the canister cover of the inhaler and thus attach the adherence monitor to the Teva Redihaler^{®} inhaler.

During normal use, the user will press down on the canister of the pMDI, which releases a pressurized mist of medicament. The patient either inhales the medicament directly or through an add-on spacer device. The adherence monitor detects when the user presses down on the canister of the pMDI and captures a timestamp of the actuation event in on board nonvolatile (NV) memory. Other data on inhalation may be collected and added to the timestamp of the event. The adherence monitor then advertises a connection using a transmission protocol such as Bluetooth Low Energy (BLE) in order to establish a link to a client device such as a smart phone. Once a BLE link is formed, the adherence monitor will send any event records (inhalations or heartbeats) to the client device for further analysis of adherence in relation to using the inhaler.

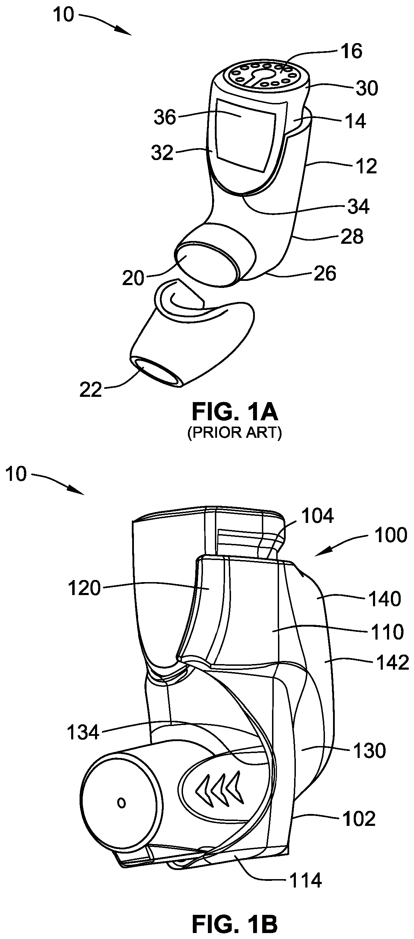

FIG. 1A is a perspective view of a known pMDI medicament inhaler 10. In this example, the inhaler 10 is a Symbicort^{®} pMDI inhaler manufactured by AstraZeneca, but the principles of the adherence monitor explained herein may be incorporated with any other similar inhalers. The inhaler 10 includes an actuator 12 that holds a pressurized medicament canister 14. The inhaler 10 includes a dose counter 16 on the top of the actuator 12. The dose counter 16 provides an indicator of the remaining doses of medicament remaining in the canister 14. The opposite end of the actuator 12 is formed into a mouthpiece 20. The mouthpiece 20 may be covered by a mouthpiece cover 22, when the inhaler 10 is not in use. The user presses down on the dose counter 16 to push the canister 14 into a firing position.

The inhaler 10 is also provided with a spray stem (not shown) extending from the canister 14, which is adapted to engage with a spray-directing element (not shown) housed within the actuator 12. When the canister 14 is pushed down into the actuator 12, the spray stem and spray-directing element combine to deliver a metered dose of medicament out through the mouthpiece 20, and into the mouth of the user. Ideally, a user will push down the canister 14 at the same time the user inhales, thus maximizing the inhalation of the medicament dose.

The mouthpiece cover 22 ensures that the mouthpiece 20 remains clean and also ensures that no foreign objects can enter the mouthpiece 20 when the inhaler is not in use. The mouthpiece cover 20 is attached via a strap 26 to the rear of the actuator 12 to ensure that the cap 24 is not inadvertently dropped or lost once it has been removed. The other end of the strap 26 is attached to a slot 28 on the rear of the actuator 12.

The dose counter 16 includes a cylindrically shaped housing 30 that is provided with a shield-shaped downward-directed surface 32 having a bottom edge 34 which abuts the top of the mouthpiece cover 22 when it is fitted onto the mouthpiece 20. The front of the shield-shaped surface 32 includes a label 36 that may include information relating to the medicament contained in the canister 14. When the mouthpiece cover 22 is on the mouthpiece 20, the shield surface 32 blocks the movement of the housing 30 of the dose counter 16 by preventing the bottom edge 34 of the downward-directed surface from downward movement, thereby preventing firing of the inhaler 10.

The downwards directed shield 32 has an aperture (not shown) that is adapted to receive a protrusion (not shown) from the top edge (not shown) of the actuator 12 in a mating relationship. When the housing 30 is depressed in order to actuate the inhaler 10, the protrusion extends through the aperture into the counter housing 30 and actuates the counter mechanism. The dose counter 16 is attached, preferably permanently, to the canister 14, in order to avoid the removal of the dose counter 16 from one canister to another canister containing a different level of medicament.

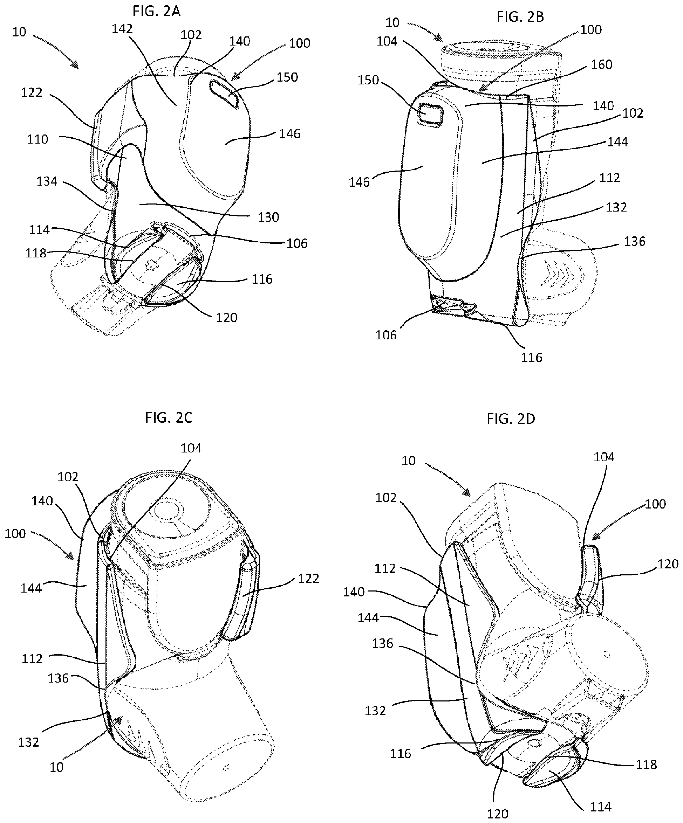

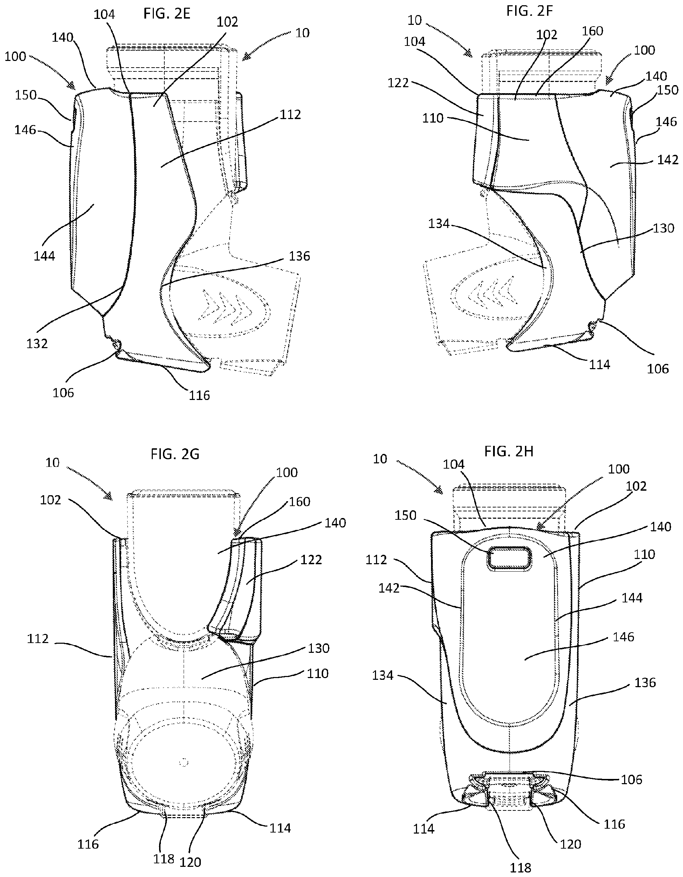

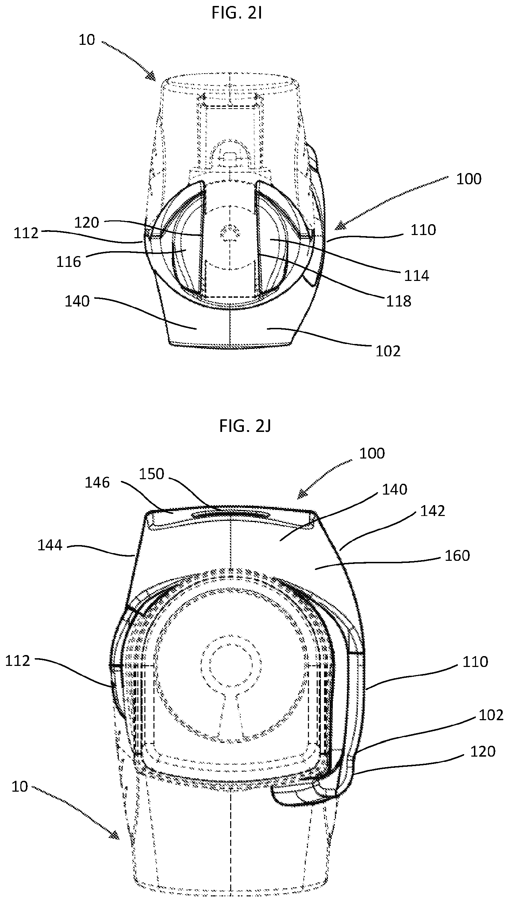

FIG. 1B is a perspective view showing the inhaler 10 mated with an adherence monitor 100 in accordance with the present invention. The adherence monitor 100 collects data as to the firing of the inhaler 10 and other useful operational data. The adherence monitor 100 attached to the inhaler 10 is shown in FIGs. 2A-2J. Thus, FIG. 2A is a perspective view of the adherence monitor 100 attached to the inhaler 10; FIG. 2B is a back perspective view of the adherence monitor 100 attached to the inhaler 10; FIG. 2C is a top perspective view of the adherence monitor 100 attached to the inhaler 10; FIG. 2D is a bottom perspective view of the adherence monitor 100 attached to the inhaler 10; FIG. 2E is a side view of the adherence monitor 100 attached to the inhaler 10; FIG. 2F is an opposite side view of the adherence monitor 100 attached to the inhaler 10; FIG. 2G is front view of the adherence monitor 100 attached to the inhaler 10; FIG. 2H is a back view of the adherence monitor 100 attached to the inhaler 10; FIG. 2I is a top view of the adherence monitor 100 attached to the inhaler 10; and FIG. 2J is a bottom view of the adherence monitor 100 attached to the inhaler 10.

The adherence monitor 100 is designed to be attached to the inhaler 10 and thus may be removed and attached to a new inhaler when the medicament supply in the canister 14 runs out. The adherence monitor 100 secures to the actuator 12 of the inhaler 10 via a housing 102 that is made of materials selected to provide mechanical strength and to be biocompatible under external, limited duration contact with the human body. The design of the housing 102 provides visual cues to guide the user to install the monitor 100 on the inhaler 10. The housing 102 includes a larger opening 104 that fits around the top of the inhaler 10. The housing 102 includes a slot 106 in the base that aligns with the slot 28 on the inhaler 10 for the cover strap 26.

The design of the housing 102 allows the mating of the adherence monitor 100 in the correct orientation relative to the inhaler, and prevents interference with the use of the inhaler 10. The wide opening 104 fits best at the top of the inhaler 10 and the narrow slot 106 at the bottom fits securely to the base of the inhaler 10. In general, the shape of the housing 102 is a reverse mold of the inhaler housing. The adherence monitor 100 can be transferred to a new inhaler by pulling it back from the inhaler 10 so that adherence monitor 100 unclips from the body of the inhaler 10.

The housing 102 of the adherence monitor 100 includes two generally curved side walls 110 and 112 that match the contours of the mouthpiece 20 of the actuator 12 of the inhaler 10. The bottom edges of the two curved side walls 110 and 112 are attached to generally semi-circular bottom projections 114 and 116. Each of the bottom projections 114 and 116 have respective edges 118 and 120 that hold the bottom of the actuator 12 of the inhaler 10. The edges 118 and 120 form a cutout to accommodate the strap 26 of the inhaler 10. The cutout formed by the edges 118 and 120 on the back of the housing 102 mimics the molded features of the inhaler 10 and serves to anchor the cover strap 26. The bottom halves of the walls 110 and 112 form respective closed ends 130 and 132 and opposite semi-circular shaped open ends 134 and 136. The open ends 134 and 136 are shaped to allow a user to hold the sides of the mouthpiece 20 of the inhaler 10. One of the side walls 110 has a top front arm 122 that extends over the front of the actuator 12 of the inhaler 10. The side walls 110 and 112 in conjunction with the projections 114 and 116 enclose the actuator 12, but leave the label 36 visible and the dosage counter 16 exposed. The projections 114 and 116 allow the mouthpiece 20 to be exposed. The slot created by the edges 118 and 120 allows placement of the strap 26. The arm 122 wraps partially around the front of the inhaler 10. The adherence monitor 100 may thus be installed on the inhaler 10 by flexing the fronts of the side walls 110 and 112 slightly and allowing the arm 122 to wrap over the front of the inhaler 10. Similarly, the inhaler 10 may be removed from the adherence monitor by moving the arm 122 away and sliding the inhaler 10 out from the side walls 110 and 112.

The side walls 110 and 112 have matching back ends that each are joined to each other and support an electronics housing 140 that forms the back of the housing 102. As will be explained below, the electronics housing 140 holds components to gather data from the operation of the attached inhaler 10. The electronics housing 140 has a pair of side components 142 and 144 that are attached to the respective side walls 110 and 112. A back panel 146 is attached to the side components 142 and 144. The combination of the back cover 146 and the side components 142 and 144 form an enclosure for the electronic components as will be explained below. The back panel 146 includes a transparent auxiliary button 150. In this example, LEDs will illuminate the auxiliary button 150. The LEDs may emit different sequences of flashes in a single color such as green. The auxiliary button 150 may also emit different colors depending on the mode of the adherence monitor 100. A semi-circular top cover 160 is formed to conform to the general shape of the actuator 12. As will be explained below, the top cover 160 creates a gap between the edge of the canister 14 when the adherence monitor 100 is engaged with the inhaler 10.

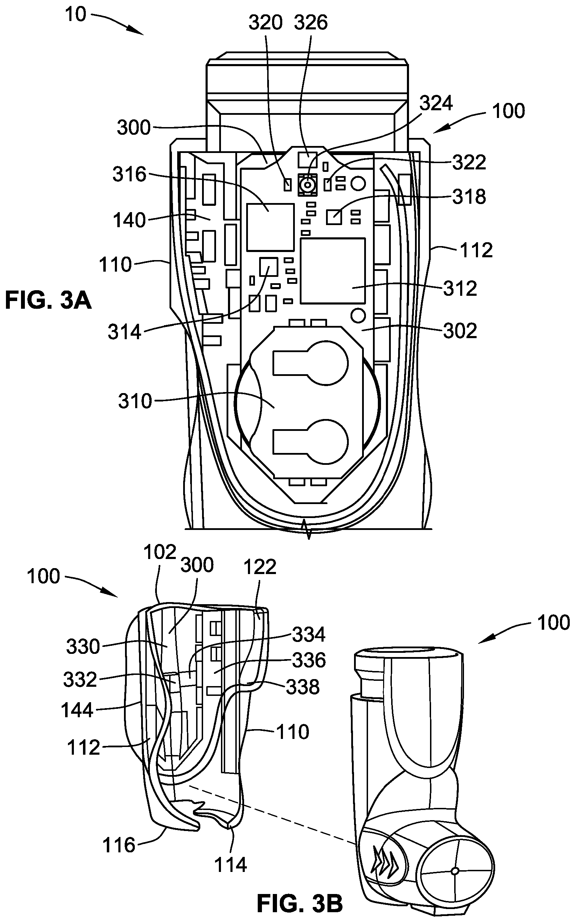

FIG. 3A is a perspective cutaway view of the inhaler 10 with the attached adherence monitor 100 showing electronic components on an internal circuit board 300 that is mounted in the electronics housing 140. The circuit board 300 has a front surface 302 having the electronic components attached by soldering or other attachment mechanisms. The circuit board 300 includes a battery 310, a piezo-electrical bender circuit 312, a piezo driver circuit 314, a communication module 316, an accelerometer 318, a pair of LEDs 320 and 322, an auxiliary switch 324, and a barometric pressure sensor 326 mounted on the front surface 302.

The battery 310 powers the electronic components on the circuit board 300. In this example, the battery 310 is a non-rechargeable (primary) coin cell battery. In this example, the intended battery life is three years of shelf life followed by one year of use life. An extra-low power inventory mode is used to preserve the battery charge during the shelf life period. Of course rechargeable batteries may be used, or other sources of power may be used.

The piezo-electric bender circuit 312 gives the user audible feedback from the use of the inhaler 10 and the adherence monitor 100. The piezo bender circuit 312 in this example provides various tones and indications (i.e. button push feedback, reminder tones, etc.). The piezo bender circuit 312 is amplified by the piezo driver 314.

In this example, the communication module 316 includes a controller that executes the algorithms to collect data and operate the adherence module 100. The controller also controls the transmission of data to a client device such as an external computing device.

In this example, the accelerometer 318 is a low power 3-axis accelerometer running all the time after exiting an inventory mode. The accelerometer 318 is mainly used for power management of the adherence monitor 100. When the sensed acceleration exceeds a predetermined threshold, the adherence monitor 100 exits sleep mode and powers up the IR sensors on the circuit board 300 as will be explained below. The accelerometer 318 also detects shaking of the inhaler 10 and the adherence module 100 with sufficient motion before inhalation detection. After a programmable amount of inactivity time, an accelerometer inactivity timer will expire and put the adherence monitor 100 into sleep mode.

In this example, the auxiliary button 150 in FIGs. 2A-2B rests above the auxiliary switch 324. The auxiliary button 150 has multiple functions including toggling reminder sounds on/off and generating manual heartbeat events. The auxiliary button 150 is captured between the cover 146 of the housing 140 and the auxiliary switch 324. This allows the user to easily press the button 150 and activate the auxiliary switch 324 to force the monitor 100 to advertise or enter various modes designed into the controller firmware. The actuator on the auxiliary button 150 is made from a transparent polycarbonate and acts as the actuator for the auxiliary switch 324 as well as a light pipe for the LEDs 320 and 322. In this example, the LEDs 320 and 322 emit a green color, and are visible through the auxiliary button 150 on the back of the cover 146. The LEDs 320 and 322 are used with various flash/strobe combinations to provide the user with feedback about the status of device operation. Both LEDs 320 and 322 have the same function and are used to create a symmetrical illumination of the auxiliary button 150.

In this example, the barometric pressure sensor 326 captures information about the inhalation of a user as the user inhales the medicament through the mouthpiece 20. The controller attaches information from the pressure sensor 326 to the event record associated with that inhalation. The additional information can include the peak, duration, total volume, and time relative to actuation. In this example, the barometric pressure sensor 326 is placed near the top of the circuit board 300, with access to the lip of the actuator 12 of the inhaler 10. When the user inhales through the mouthpiece 20 of the inhaler 10, the pressure reading changes measurably and quantifiably, providing data on the profile of the inhalation. The barometric pressure sensor 326 may provide pressure data that may be used to calculate the duration of the inhalation (as well as if an inhalation did not take place, as in a priming event). The data from the pressure sensor 326 may be used to capture the peak value, which is a pressure change measurement between atmospheric pressure and peak pressure drop experienced. This is not a calibrated flow rate. The data from the pressure sensor 326 may also be used to calculate the total volume of air inhaled during the dosing event through the sum of instantaneous flow rates over time. Finally, the data from the pressure sensor 326 may be used to associate the beginning of the inhalation with the actuation of the inhaler 10, indicating when the medicament was released relative to the initiation of the breath. This inhalation data is then attached to the nearest appropriate actuation event(s). In this way, it is possible to have a single inhalation associated with multiple actuations, or a single actuation. In the event that an actuation is not recorded and a pressure drop is recorded, indicating an inhalation event, the data related to the inhalation event is not captured as a usage event. The data may be transferred with the next heartbeat event or during another event. In this example, the pressure sensor 326 is only activated when motion has been detected, reducing battery consumption.

FIG. 3B is a perspective view of a rear surface 330 of the circuit board 300. The rear surface 330 generally includes electrical traces for connecting the components on the front surface 302 of the circuit board 300 shown in FIG. 3A. The rear surface 330 includes an inhaler attachment detection Infrared (IR) sensor 332. The rear surface 330 also includes a socket 334 that attaches to one end of a flexible cable 336. The other end of the flexible cable 336 attaches to a remote medicament actuation detection IR sensor 338. The actuation detection IR sensor 338 is located in proximity to the shield 32 of the inhaler 10 when the adherence monitor 100 is attached to the inhaler 10. The cable 336 is wrapped around the inner surface of the side wall 110 to be located near the front of the arm 122 in proximity to the edge 34 of the shield 32. The cable 336 is protected by a separate cover. In this example, the inhaler attachment detection IR sensor 332 and actuation detection IR sensor 338 are identical IR sensor circuits.

The IR sensors 332 and 338 each have an infrared emitter and infrared receiver, pointed in the same direction. The receiver cannot sense the light from the emitter unless the emitted light is reflected off another surface. The process of determining proximity to the IR sensor involves taking a powered reading, where the infrared emitter is turned on and the receiver value is collected. Second, an unpowered reading is taken, where the infrared emitter is off and the receiver value is collected. The difference of these two values is used in determining the proximity of an object to the sensor. If the difference is high, then it means something is reflecting the IR light onto the receiver, indicating proximity. If the difference is low, then nothing is reflecting the IR light onto the receiver, indicating an open space. However, it may be recognized that different types of IR sensors may be used. Further, other types of sensors may be used to detect attachment and actuation consistent with the below description.

The remote primary medicament actuation detection IR sensor 338 is used to determine when the user depresses the canister 14 of the inhaler 10. The actuation detection IR sensor 338 detects the proximity of the shield 32 when the canister 14 is depressed. The actuation detection IR sensor 338 is positioned to correspond to a position where the shield 32 will be located when the canister 14 is depressed sufficiently to release a dose of the medicament. The shield 32 will be pushed in a downward motion, thus moving in front of the actuation detection sensor 338.

The inhaler attachment detection IR sensor 332 is used to determine whether or not the inhaler 10 is attached to the adherence monitor 100. As shown in FIG. 3B, the inhaler attachment detection IR sensor 332 is placed on the circuit board 300 along the midline of the main body of the inhaler 10 and will detect when the adherence monitor 100 has been fully seated relative to the inhaler 10.

The inhaler attachment detection IR sensor 332 is powered from a separate microcontroller general purpose input/output (GPIO) pin from the communication module 316 so that it does not drain the battery 310 prematurely. Powering the inhaler detection IR sensor 332 through a separate pin allows sampling from the inhaler attachment detection IR sensor 332 in the case where the adherence monitor 100 has been installed for some time on the inhaler 10 without a static power draw.

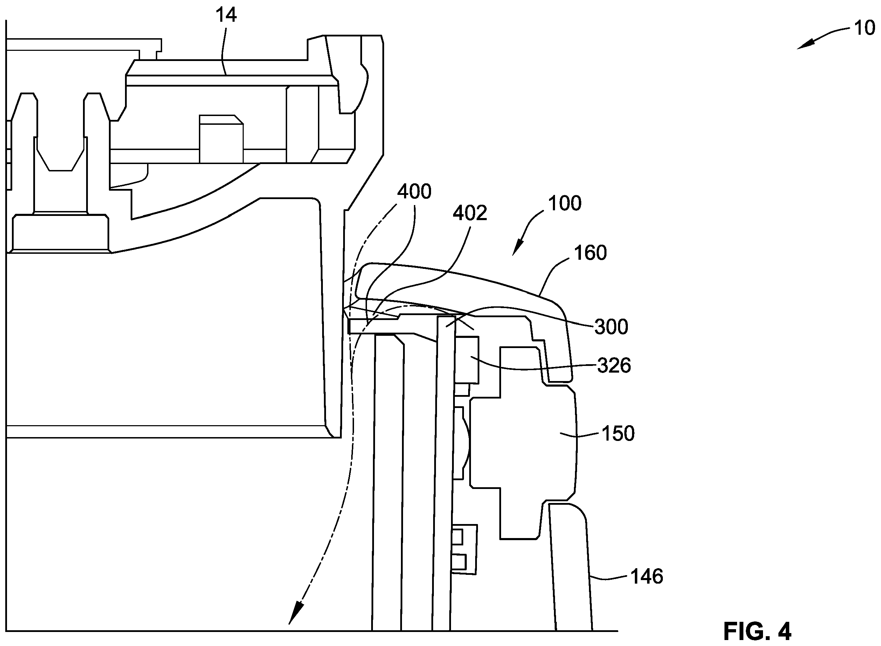

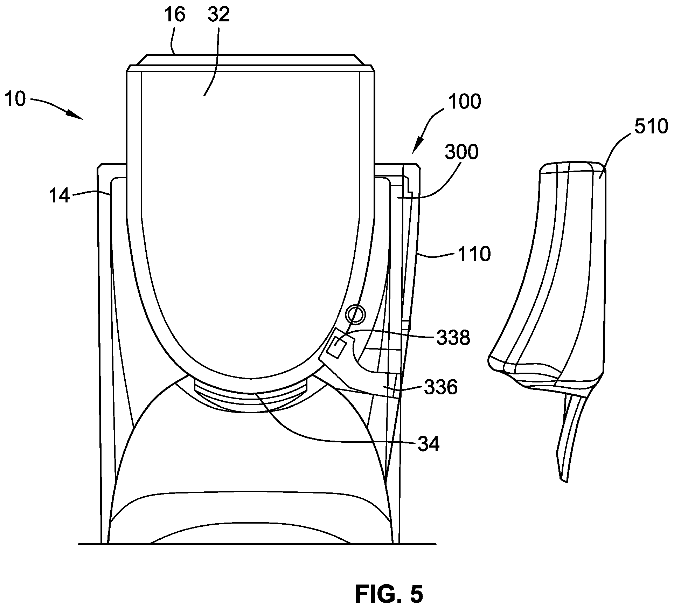

The operation of the adherence monitor 100 is explained in relation to FIGs. 4 and 5. FIG. 4 is a cross section view of the canister 14 and actuator 12 of the inhaler 12 in relation to the attached adherence monitor 100. FIG. 5 is a front side cross section view of the shield 32 of the dosage counter 12 in relation to the circuit board 300 of the adherence monitor 100. The event detection algorithm executed by the controller of the adherence monitor 100 relies on signals from the accelerometer 318 detecting movement of the inhaler 10, the inhaler attachment detection infrared sensor 332 detecting whether the adherence monitor 100 is attached to the inhaler 10, and the actuation detection IR sensor 338 detecting release of medicament from the canister 14. Thus, an event is detected when a sequence of signals is received, first from the accelerometer 318 detecting movement of the inhaler, then the inhaler attachment detection IR sensor 332 indicating the adherence monitor 100 is attached to the inhaler, and finally, the signal from the inhaler actuation detection sensor 338 indicating release of the medicament for a sufficient amount of time. The barometric pressure sensor 326 is used to provide additional information indicating if an inhalation occurred with the release of the medicament. The accelerometer 318 can also be used to detect if the user of the inhaler 10 shook the inhaler 10 prior to releasing a dose of the medicament. When the inhaler 10 and attached adherence monitor 100 is moved, the accelerometer 318 is used to wake the controller from sleep mode (a low power state).

When the user inhales through the mouthpiece 20 of the inhaler 10, air flow represented by a dashed line 400 is pulled through the inhaler between the actuator 10 and body 12. The cover 160 of the adherence monitor is designed to slightly impede this airflow and create a pressure drop through a gap 402 between the cover 160 of the adherence monitor 100 and the canister 14 of the inhaler 10. The barometric pressure sensor (BPS) 326 registers the pressure drop during the inhalation and this signal is recorded in the event record by the controller.

The IR sensor 338 attached to the flex cable 336 is used to detect when the shield 32 passes by the IR sensor 338 indicating that a dose of the medicament has been released from the inhaler 10. As explained above, the flex cable 336 follows the interior surface of the side wall 110 and is generally extended by the arm 122. As shown in FIG. 5, a cable cover 510 is generally installed over the cable 336 to protect the cable 336 and the IR sensor 338. When the user presses down on the dosage counter 16, the canister 14 of the inhaler 10 moves in a downward direction. The shield 32 therefore moves in front of the actuation detection IR sensor 338. The IR sensor 338 thus detects the movement of the canister 14 by detecting the presence and absence of the shield 32. The controller of the adherence monitor 100 on the communication module 316 will record a timestamp associated with the RTC and store an inhalation event in the event queue. If the inhalation pressure data is available from the pressure sensor 326, this will also be stored in the inhalation event

In this example, once the adherence monitor 100 detects that the inhaler 10 has been actuated and stores the information in the event queue, the adherence monitor 100 makes an attempt to offload the unsent items in the event queue to a client device such as a mobile device. In this example, this transmission takes place via the BLE wireless protocol implemented inside the communication module 316. The client device indicates success or failure of the event transmission, which is then communicated back to the controller.

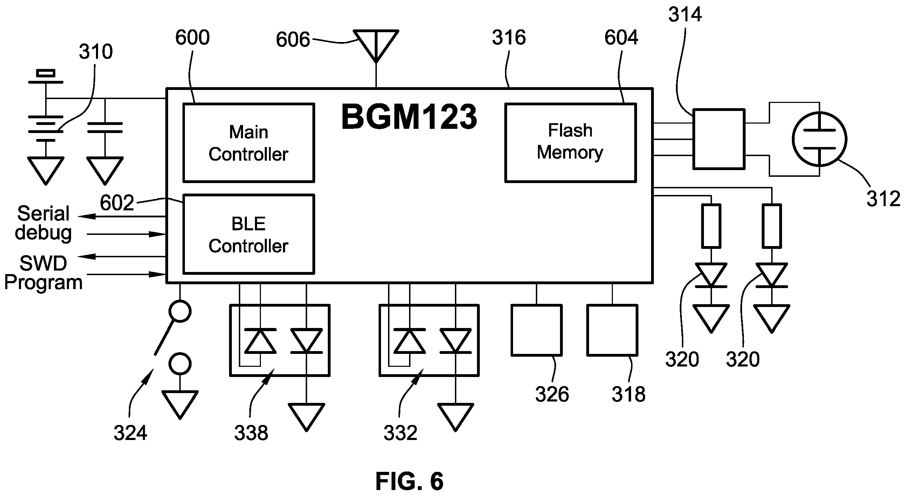

FIG. 6 is a circuit diagram of the components on the printed circuit board 300. As explained above the communications module 316 serves as the controller for the inventive adherence monitor 100. The communication module 316 includes a main controller 600 and communication microcontroller 602. The communication module 316 includes a memory 604 that is used for firmware as well as data storage. In this example the communication module 316 is a BGM123 Bluetooth Low Energy (BLE) system in package (SIP). Of course, any suitable component or set of components with appropriate functionality may be used for the communication module 316. In this example, the BGM123 BLE SIP includes an ARM M4 microcontroller (main controller 600) and an ARM M0 microcontroller (communication microcontroller 602) that run the internal Bluetooth Smart^{™} compliant stack. In this example, the BGM123 BLE SIP acts as both the Bluetooth radio and the main microprocessor of the adherence monitor 100. In this example, the memory 604 is embedded flash memory in the BGM123 BLE SIP that stores the firmware that is executed on the controller 600. A portion of this flash memory is also used to store the event records (inhalation & heartbeat) that are detected by the various sensors.

The BGM123 BLE SIP also contains embedded SRAM memory used for temporary scratch space and data structures. Several peripherals (UART, SPI, A2D, RTC, PWM, DMA, and power management) are utilized to drive the system. The BGM123 BLE SIP is driven directly from the onboard battery 310 with no other power regulation other than its internal DC-DC switcher which it uses to improve the energy efficiency of the system. The module contains an integrated omni-directional chip antenna 606 that allows transmission of the collected data to an external client device.

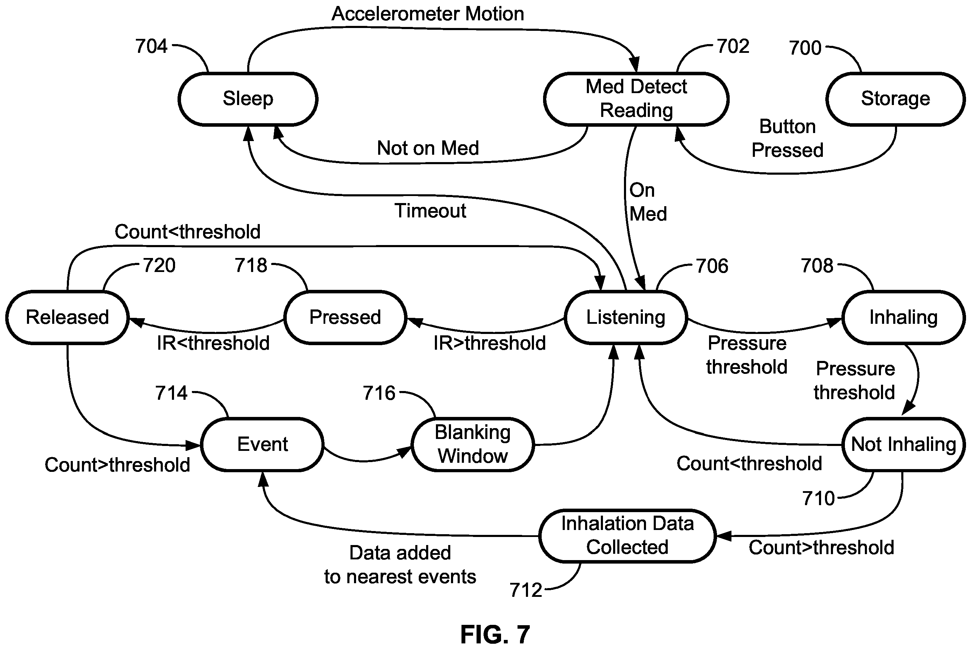

FIG. 7 is a state diagram of the operational event detection routine executed by the controller 600 of the adherence monitor 100 in accordance with the invention. A storage state (also known as an inventory mode) 700 is the lowest power mode in which the accelerometer 318 is turned off, the internal clock is not running, and the adherence monitor is not advertising (transmitting or receiving over the Bluetooth radio). In this lowest power state the only way to wake the adherence monitor 100 is to press the button 150. Users will first receive the adherence monitor 100 in the storage state or inventory mode 700, but after their first interaction with it, the monitor 100 will only return to a sleep state 704 and will not return to the storage state 700. A signal from the accelerometer 318, indicating shaking of the inhaler 10, results in proceeding to the inhaler attachment detection reading state 702.

When the button 150 is pressed, the routine moves to the inhaler attachment detection reading state 702. If no signal from the inhaler attachment detection sensor 332 is received, the adherence monitor 100 is not attached to the inhaler 10, and the routine moves to the sleep state 704. The sleep state 704 is a low power mode where the sensors and components are on low power. When a signal from the inhaler attachment detection sensor 332 is received indicating the attachment of the adherence monitor 100 to the inhaler 10, the routine moves to a listening state 706. The listening state 706 listens for data from the primary medication actuation IR sensor 332. Separately, the barometric pressure sensor 326 is polling and trying to detect an inhalation. If the signal from the inhalation attachment detection sensor 332 is not detected, the routine moves back to the sleep state 704.

In the listening state 706, if the pressure detected by the pressure sensor 326 is less than a threshold value, the routine shifts to an inhaling state 708. If the pressure detected by the pressure sensor 326 is greater than the threshold value, the routine shifts to a not inhaling state 710. The routine shifts back to the listening state 706 if the count is less than the threshold value. If the count is greater than the threshold, the routine shifts to an inhalation data collected state 712. After a time stamp is applied to the nearest events, the routine shifts to an event state 714. The event state 714 indicates that a dose of the medicament has been released. The routine then shifts to a blanking window state 716 and then to the listening state 706. The blanking window state 716 is used to prevent double tapping of the inhaler 10, or accidental detection of a second dose immediately following the release of medicament from the canister 14. The routine avoids detecting multiple events in error from the slight shifts that occur during an actuation. This prevents the blanking window, which is a fraction of a second, from detecting more than the correct number of events. A return is made to the listening state 706 after detection of a dose because it is possible to take multiple doses and the routine cannot rely on a user moving the inhaler 10 enough to trigger the accelerometer 318 again to repeat the full cycle of detection.

In the listening state 706, if the output of the actuation sensor 338 is greater than a threshold value indicating actuation, the routine shifts to a canister pressed state 718. If the output signal from the actuation sensor 338 is less than the threshold value, indicating the actuation of the canister 14 is over, the routine shifts to a released state 720. The routine then shifts to the event state 714 if the count is greater than the threshold value. If the count is less than the threshold value, the routine shifts to the listening state 706.

Usage events contain a timestamp captured from the moment that the actuation detection IR sensor 338 detects actuation. The duration of the press down of the canister 14 during actuation is also added to the usage event. If the accelerometer 318 detects shaking prior to actuation, the shake intensity and shake duration are added to the usage event. If the barometric pressure sensor 326 detects an inhalation around the time of actuation, either before, during, or after, the peak value of the pressure measurement, the duration of the inhalation, and the time between actuation and start of inhalation are added to data related to the usage event. All usage events will also include a battery measurement and temperature measurement captured from the controller on the communications module at the time of actuation.

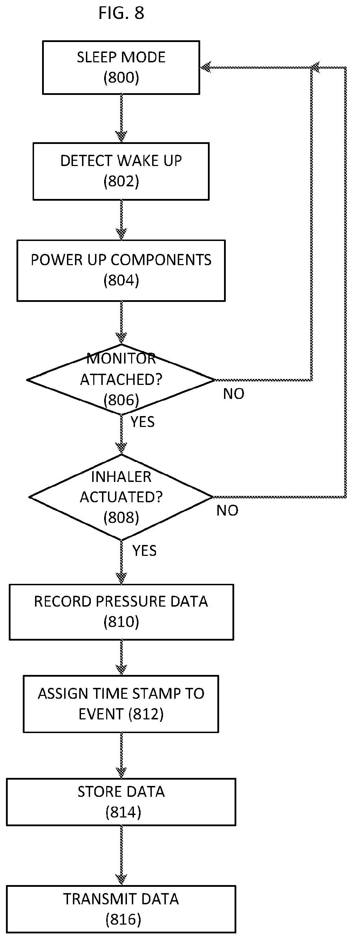

FIG. 8 is a flow diagram of the inventive routine executed by the controller 600 to record actuation events of the inhaler 10. The flow diagram in FIG. 8 is representative of example machine readable instructions for collecting and analyzing adherence data collected from the inventive adherence monitor 100 in FIG. 1B. In this example, the machine readable instructions comprise an algorithm for execution by: (a) a processor; (b) a controller; and/or (c) one or more other suitable processing device(s). The algorithm may be embodied in software stored on tangible media such as flash memory, CD-ROM, floppy disk, hard drive, digital video (versatile) disk (DVD), or other memory devices. However, persons of ordinary skill in the art will readily appreciate that the entire algorithm and/or parts thereof can alternatively be executed by a device other than a processor and/or embodied in firmware or dedicated hardware in a well-known manner (e.g., it may be implemented by an application specific integrated circuit [ASIC], a programmable logic device [PLD], a field programmable logic device [FPLD], a field programmable gate array [FPGA], discrete logic, etc.). For example, any or all of the components of the interfaces can be implemented by software, hardware, and/or firmware. Also, some or all of the machine readable instructions represented by the flowcharts may be implemented manually. Further, although the example algorithm is described with reference to the flowchart illustrated in FIG. 8, persons of ordinary skill in the art will readily appreciate that many other methods of implementing the example machine readable instructions may alternatively be used. For example, the order of execution of the blocks may be changed, and/or some of the blocks described may be changed, eliminated, or combined.

In general, the components are maintained in the sleep state 704 in FIG. 7 to preserve power after the adherence monitor 100 is activated from the storage/inventory state (800). The routine detects whether the monitor 100 should be activated to expect data from either the button 150 being pressed or from a signal from the accelerometer 318 (802). If the system is woken up, the controller 600 powers up the components on the adherence monitor (804). The system determines whether the adherence monitor 100 is attached to the inhaler 10 by reading the output of the attachment detection sensor 332 (806). The routine loops back to the sleep state (800) if no inhaler is attached. If an inhaler is attached, the routine continuously determines whether the inhaler 10 has been actuated by reading the actuation detection sensor 338 (808). If the inhaler has not been actuated within a predetermined period of time, the routine returns to the sleep state (800). If the inhaler has been actuated, the controller 600 detects the actuation via a signal from actuation detection sensor 338 (810). Alternatively, the actuation may be based on detection of a reading from the pressure sensor 326. The inhalation may also be based on a signal from either the actuation detection sensor 338 or the pressure sensor 326 or requiring a signal from both of them.

The routine collects inhalation data in the form of pressure data from the pressure sensor 326 and attaches the inhalation data to the actuation event with a time stamp (812). The inhalation data, time stamp, and event data are stored to the memory (814). The related inhalation data and actuation event data then is transmitted to the external device (816).

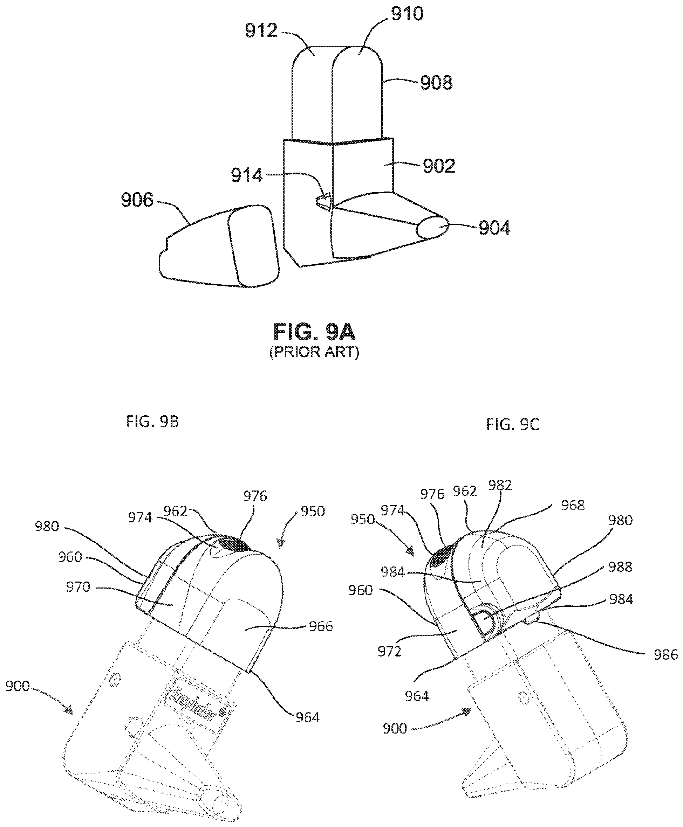

FIG. 9A shows a perspective view of another example known prior art inhaler 900 that may be attached to a modular adherence monitor incorporating the principles explained above. In this example, the inhaler 900 is an Orion Easyhaler^{®} inhaler. The inhaler 900 includes a square shaped actuator body 902 having a conically shaped mouthpiece 904. A dust cap 906 is provided to protect the mouthpiece 904 when the inhaler 900 is not in use. A canister cover 908 is fit over a canister (not shown) that is inserted within the body 902. The body 902 has an open top end that allows the insertion of the canister and the attached canister cover 908. The canister cover 908 may be depressed relative to the actuator body 902 to put pressure on the canister to emit a dose of the stored medicament. The top of the canister cover 908 includes a button 910 and a series of vents 912. The actuator body 902 includes a dosage counter 914.

The operation of the inhaler 900 is initiated by a user shaking the inhaler 900 up and down several times. The user will hold the inhaler 900 upright and pressing down once on the button 910 with the forefinger until a click is heard. The user then releases their forefinger and the medicament dose from the canister is ready. The user breathes out as far as is comfortable, away from the inhaler 900. The patient then puts the mouthpiece 904 in their mouth while sealing their lips around the mouthpiece 904. The patient breathes in as quickly and deeply as possible until their lungs are full.

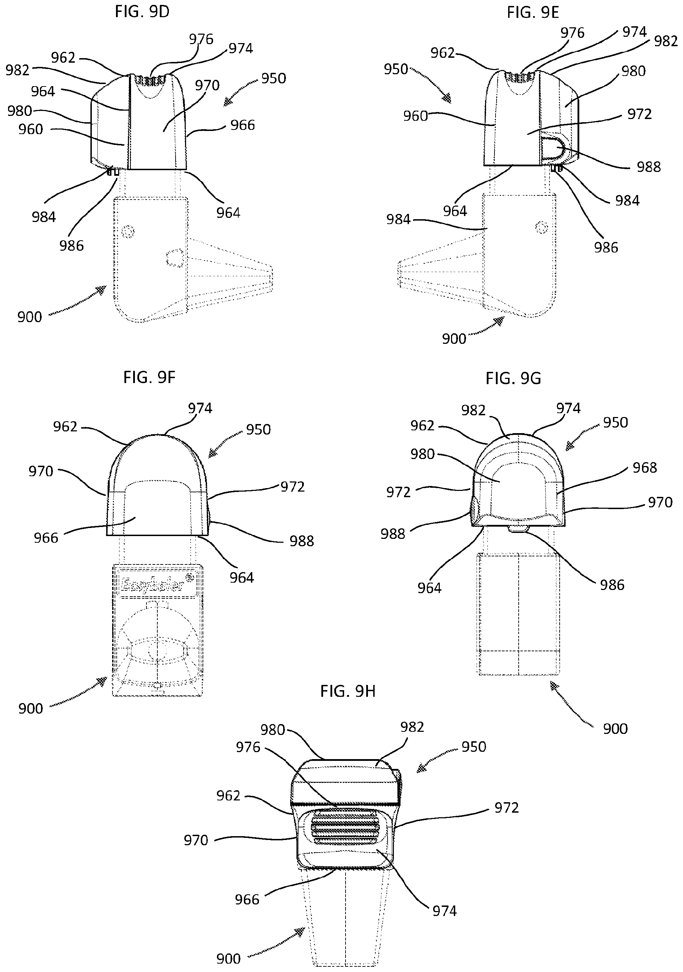

FIG. 9B is a front perspective view of the inhaler 900 in FIG. 9A with an example of an attached modular adherence monitor 950, which is described below for clarifying only. FIG. 9C is a rear perspective view of the modular adherence monitor 950 attached to the inhaler 900. FIG. 9D is a side view of the modular adherence monitor 950 attached to the inhaler 900. FIG. 9E is an opposite side view of the modular adherence monitor 950 attached to the inhaler 900. FIG. 9F is a front view of the modular adherence monitor 950 attached to the inhaler 900. FIG. 9H is a rear view of the modular adherence monitor 950 attached to the inhaler 900. FIG. 9G is a top view of the modular adherence monitor 950 attached to the inhaler 900.

As will be explained, the adherence monitor 950 may record actuation events and corresponding inhalation data from the inhaler 900. The adherence monitor 950 includes a support body 960 that has a closed top end 962 and an opposite open end 964. The support body 960 has a front wall 966 and an opposite rear wall 968. Two side walls 970 and 972 are joined to the front wall 966 and rear wall 968. The shape of the support body 960 is designed to fit over and around the canister cover 908 of the inhaler 900 as shown in FIGs. 9B-9H by inserting the open end 964 over the canister cover 908.

The top of the walls 966, 968, 970 and 972 are enclosed by a top member 974. The top member 974 has a series of features 976 that mimic the tactile feel of the vent features of the inhaler 900. An electronics housing 980 snaps to the rear wall 968 and protects the electronic components. The top of the electronics housing 980 includes a curved section 982 that is joined with the top member 974. The bottom of the electronics housing 980 is open and forms a gap 984 in conjunction with the body 902 of the inhaler 900. A contact member 986 protrudes from the gap 984. As will be explained below, the monitor 950 is inserted over the canister cover 908. Thus, when the patient pushes down on the monitor 950, the canister cover 908 is pushed into the body 902 to release a dose of the medicament from the canister. When the monitor 950 is pushed down, the contact member 986 contacts the edge of the body 902. The side wall 972 also includes an auxiliary button 988.

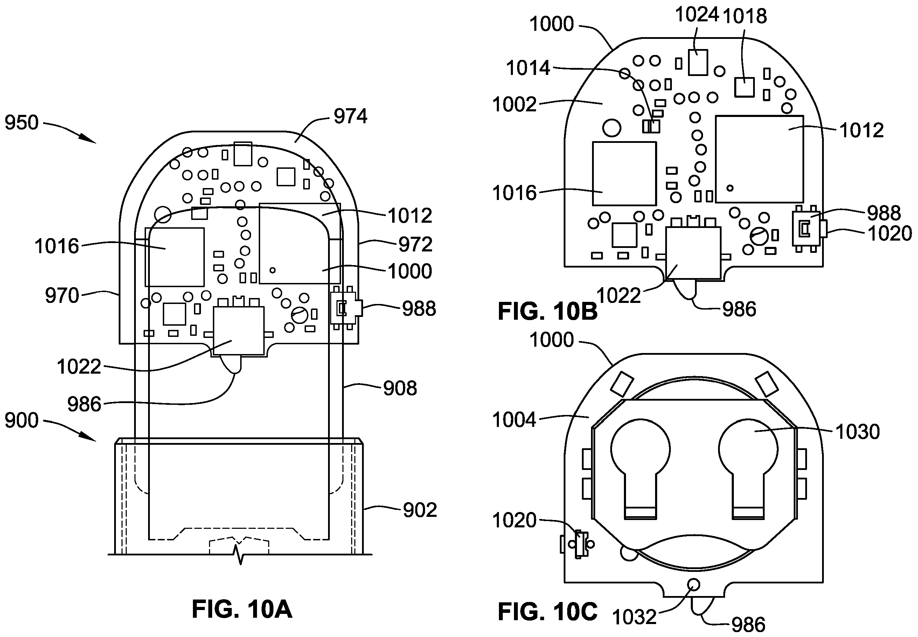

FIG. 10A is a cutaway rear view of the adherence monitor 950 inserted over the canister cover 908 of the inhaler 900. FIG. 10A shows an internal circuit board 1000 that is mounted in the electronics housing 980. The circuit board 1000 is mounted so it is parallel to the rear of the canister cover 908 when the adherence monitor 950 is inserted over the canister cover 908. FIG. 10B shows a close up view of the front surface 1002 of the circuit board 1000. The front surface 1002 is in proximity to the rear wall 968 of the monitor 950. FIG. 10C shows a close up view of a rear surface 1004 of the circuit board 1000. The rear surface 1004 is in proximity to the interior of the housing 980.

The front surface 1002 of the circuit board 1000 has electronic components attached by soldering or other attachment mechanisms. The circuit board 1000 mounts a piezo-electrical bender circuit 1012, an attachment detection IR sensor 1014, a communication module 1016, an accelerometer 1018, an auxiliary switch 1020, a limit switch 1022 and a barometric pressure sensor 1024 mounted on the front surface 1002. The circuit board 1000 includes a battery 1030 and an LED 1032 mounted on the rear surface 1004. The LED 1032 is vertically mounted so that the light is directed out of the side of the circuit board 1000 and through the auxiliary button 988. The auxiliary button 988 in this example is clear plastic, so the light from the LED 1032 illuminates the plastic button 988, providing feedback to the user. In this example, the communication module 1016 is a BGM123 BLE SIP chip with the capabilities and functions explained above in reference to FIG. 6.

The battery 1030 powers the electronic components on the circuit board 1000. In this example, the battery 1030 is a coin cell battery. The adherence monitor 950 has an extra-low power inventory mode is used to preserve the battery charge during the shelf life period. Of course rechargeable batteries may be used, or other sources of power may be used.

The piezo-electric bender circuit 1012 gives the user audible feedback from the use of the inhaler 900 and the adherence monitor 950. The communication module 1016 is similar to the communications module 316 in FIG. 3 and includes a controller that executes the algorithms to collect data and operate the adherence module 950. The controller also controls the transmission of data to a client device such as an external computing device.

In this example, the accelerometer 1018 is a low power 3-axis accelerometer running all the time after exiting an initial storage/inventory mode. The accelerometer 1018 is mainly used for power management of the adherence monitor 950 similar to the accelerometer 318 explained above. The accelerometer 1018 also detects shaking of the inhaler 900 and the adherence module 950 with sufficient motion before actuation detection.

In this example, the auxiliary button 988 in FIGs. 9C and 9E rests above the auxiliary switch 1020. The auxiliary button 988 has multiple functions including toggling reminder sounds on/off, generating manual heartbeat events, and waking the monitor 950 from the inventory state. In this example, the LED 1032 emits a green color, and is used with various flash/strobe combinations to provide the user with feedback about the status of device operation by illuminating the button 988.

In this example, the barometric pressure sensor 1024 captures information about the inhalation of a user as the user inhales the medicament through the mouthpiece 904 similar to the pressure sensor 326 explained above. The controller in the communication module 1016 attaches information from the pressure sensor 1024 to the event record associated with an actuation event of the inhaler 900. The additional information can include the peak, duration, total volume, and time relative to actuation. In this example, the barometric pressure sensor 1024 is placed near the top of the circuit board 1000, with access to an air reservoir created by an internal gap between the interior of the adherence monitor 950 and the canister cover 908 of the inhaler 900. When the user inhales through the mouthpiece 904 of the inhaler 900, the pressure reading changes measurably and quantifiably, providing data on the profile of the inhalation. The output from the barometric pressure sensor 1024 may provide pressure data that may be used to calculate the duration of the inhalation (as well as if an inhalation did not take place, as in a priming event). The data from the pressure sensor 1024 may be used to capture the peak value, which is a pressure change measurement between atmospheric pressure and peak pressure drop experienced.

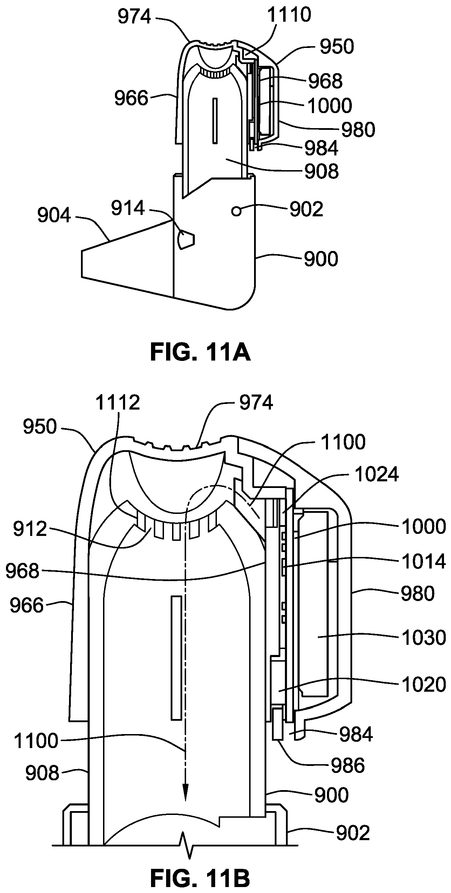

FIG. 11A is a cross-section side view of the inhaler 900 with the attached adherence monitor 950. FIG. 11B is a close-up cutaway side view of the inhaler 900 and the attached circuit board 1000. The controller in the communication module 1016 operates the adherence monitor 950 according to the routine explained above with reference to FIG. 8. A user pushes the top member 974 of the adherence monitor 950, which causes the canister cover 908 to be pressed down. When the canister cover 908 is pressed down into the body 902 of the inhaler 900, the contact member 986 attached to the limit switch 1022 is tripped when it contacts the edge of the open end of the body 902 of the inhaler 900.

Tripping the limit switch 1022 thus indicates actuation of the inhaler 900. The signal from the limit switch 1022 is processed and appended to the event information indicating when the canister cover 908 was pressed relative to the start of the inhalation. The location of the circuit board 100 allows the placement of the limit switch 1022 that is a set distance (e.g., 1 cm) from the body 902 when the canister cover 908 is at rest. At this distance, the contact member 988 reliably trips the limit switch 1022 by contacting the edge of the body 102 each time the canister cover 908 is depressed.

The vents 912 cut into the top of the canister cover 908 are key to detecting the inhalation. When the patient inhales through the inhaler 900, air is pulled through multiple vents in the inhaler as shown by the orange dashed lines 1100 in FIG. 11B. In order to generate a stable signal during the inhalation, a small reservoir of air 1110 is designed into the top of the adherence monitor 950. The reservoir 1110 is bounded by the top surface 974 that is spaced from the top of the canister cover 908 when the adherence monitor 950 is seated on the canister cover 908. A series of hard plastic ribs 1112 protrude from the interior of the top member 974 and have two functions in this example. One function is to increase the height of the adherence monitor 950 in order to add a slope to the electronics cover 980. The slope is necessary to reduce the risk of the user of the inhaler 900 jamming the canister cover 908 in the inhaler body 902 by pressing down off-center. The second function of the ribs 1112 is to increase the size of the air reservoir 1110 between the canister cover 908 and the adherence monitor 950

The barometric pressure sensor 1024 sits in the air reservoir 1110 to detect the pressure drop during the inhalation of the medicament from the inhaler 900. The barometric pressure sensor 1024 is activated when the user presses down on the adherence monitor 950 and canister cover 908. When the pressure sensor 1024 is activated, it begins "listening" for a pressure drop indicating that an inhalation has begun. Thresholds are set within the firmware algorithm executed by the controller in the communication module 1016 to filter out false signals.

The attachment detection IR sensor 1014 mounted on the front surface 1002 of the circuit board 1000 detects when the adherence monitor 950 is attached to the inhaler 900. Similar to the adherence monitor 100, the adherence monitor 950 is primarily kept in a low-power sleep state. The accelerometer 1018 is used to wake the monitor 950 from this mode and to detect shaking of the inhaler 900. When the adherence monitor 950 is awakened, the infrared sensor 1014 gates further sensing depending on the state of the device. If the sensor 1014 detects a surface in proximity, indicating that the adherence monitor 950 is attached to the inhaler 900, further sensing mechanisms are activated.

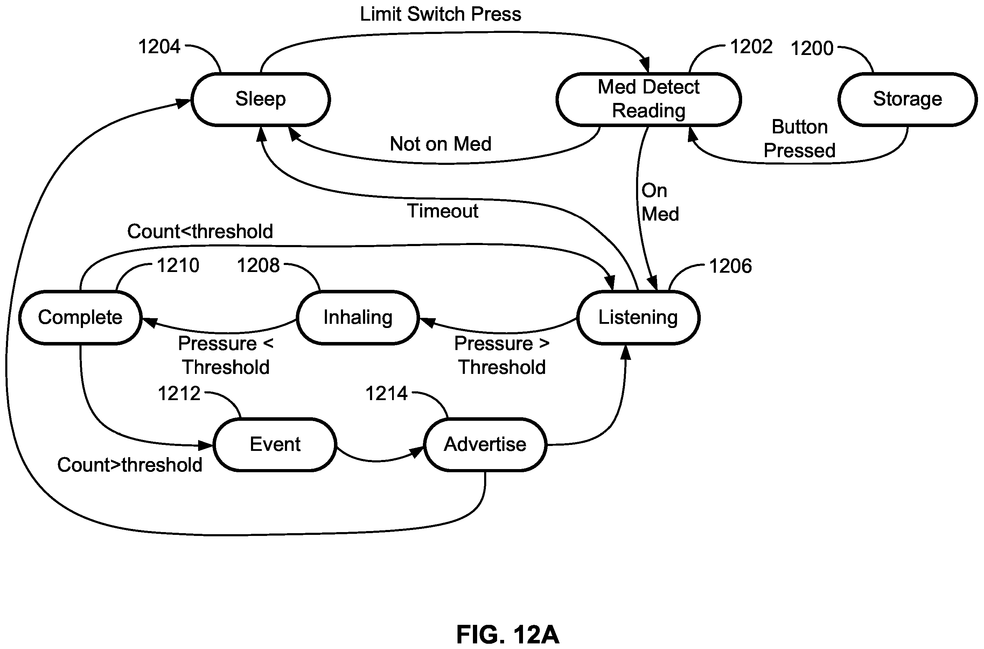

FIG. 12A is a state diagram for usage detection of the inhaler 900. A storage state (also known as an inventory mode) 1200 is the lowest power mode in which the accelerometer 1018 is turned off, the internal clock is not running, and the adherence monitor is not advertising. In this lowest power state the only ways to wake the adherence monitor 100 to proceed to a detection state 1202 is to either press the auxiliary button 988 and thereby activate the limit switch 1020 or by pushing down on the canister cover 908 and thereby activate the limit switch 1022. Users will first receive the adherence monitor 950 in the storage state or inventory mode 900, but after their first interaction with it, the monitor 950 will only return to a low-power sleep state 1204 and will not return to the storage state 1200.

After the auxiliary button 988 is pressed to exit from the storage/inventory state 1200 and proceed to the detection state 1202, the attachment detection IR sensor 1014 is tested. The signal from the attachment detection IR sensor 1014 is measured to determine if the adherence monitor 950 is attached to the inhaler 900. If it is determined that the monitor 950 is not attached to the inhaler 900, then the monitor 950 proceeds to the sleep state 1204. If the monitor 950 is attached to the inhaler 900, then the controller proceeds to a listening state 1206 where the barometric pressure sensor 1024 is activated and a timer started. In this example, the pressure sensor 1024 starts collecting data at a rate of 10Hz, maintaining a moving average of the ambient pressure. When the pressure varies from the moving average by more than a configurable pressure activity threshold, an inhaling state 1208 is entered and the moving average is fixed at its current value. As long as the pressure sensor value stays at least a configurable hysteresis value underneath the pressure activity threshold value, the algorithm stays in the inhaling state 1208. After the pressure returns below the lower threshold, and if the number of inhalation samples is greater than a configurable duration threshold, then an inhalation complete state 1210 is entered. The inhalation event is recorded in an event state 1212. The monitor 950 then starts advertising the inhalation event 1214 and returns to the sleep state 1204. If the measured duration is less than the duration threshold, then the algorithm returns to the listening state 1206. If, after a configurable timeout period after the limit switch 1022 is pressed, no event has been detected, the algorithm stops measuring for an event and returns to the sleep state 1204.

Usage events contain a timestamp captured from the moment that the actuation detection IR sensor detects actuation. The duration of the press during actuation is also added to the usage event. If the accelerometer detects shaking prior to actuation, the shake intensity and shake duration are added to the usage event. If the barometric pressure sensor detects an inhalation around the time of actuation, either before, during, or after, the peak value of the pressure measurement, the duration of inhalation, and the time between the start of inhalation to the peak of inhalation are added to the usage event. All usage events will also include a battery measurement and temperature measurement captured from the controller at the time of actuation.

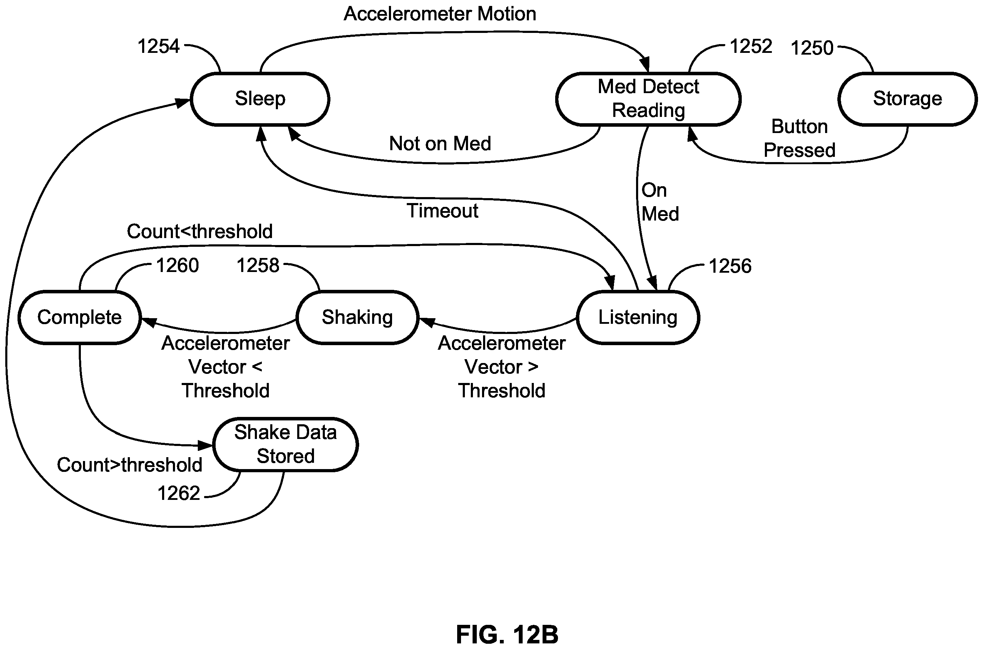

FIG. 12B is a state diagram for the shake detection. Accelerometer motion is monitored and when the motion reaches a pre-determined threshold indicating a shake has occurred, the amount of samples above the shake threshold are counted and the data is appended to the inhalation event packet. Shake data captured will expire if it does not occur within a specified time frame leading up to an inhalation event.

The controller in the communication module 1016 runs the shake detection algorithm. The controller starts in a low-power storage mode 1250. As explained above, pressing the auxiliary button 988 thereby activating the limit switch 1022 by pushing down on the canister cover 908 causes the routine to exit the low-power storage mode 1250 and proceed to a monitor detection state 1252. After waking from the storage/inventory state 1200, the attachment detection IR sensor 1014 is measured to determine if the adherence monitor 950 is attached to the inhaler 900. If it is not, the monitor goes into a low-power sleep state 1254. After the auxiliary button 988 is pressed, the attachment detection IR sensor 1014 is tested. If it is determined that the monitor 950 is not attached to the inhaler 900, then the monitor 950 returns to the sleep state 1254. If the monitor 950 is attached to the inhaler 900, then the controller proceeds to a listening state 1256

If the monitor 950 is attached to the inhaler 900, then the accelerometer 1018 is configured for active measurements and a timer started in the listen state 1256. When the magnitude of the acceleration vector is more than a configurable motion activity threshold, a shaking state 1258 is entered. As long as the accelerometer vector magnitude does not go below the configurable motion activity threshold value for a configurable number of samples, the algorithm will continue to measure the accelerometer in the shaking state 1258. After the accelerometer vector magnitude returns below the activity threshold, and if the number of shaking samples is greater than a configurable threshold of shaking samples, then the shaking is considered complete and the routine moves to a complete state 1260. The shaking data is then stored 1262 and later included in the next detected usage event. The algorithm then returns to the sleep state 1254. If the measured number of shaking samples is less than the threshold of shaking samples, then the monitor 950 returns to the listening state 1256. If a configurable timeout period after the accelerometer motion is detected occurs with no shaking detected, the algorithm stops measuring for shaking and returns to the sleep state 1254.

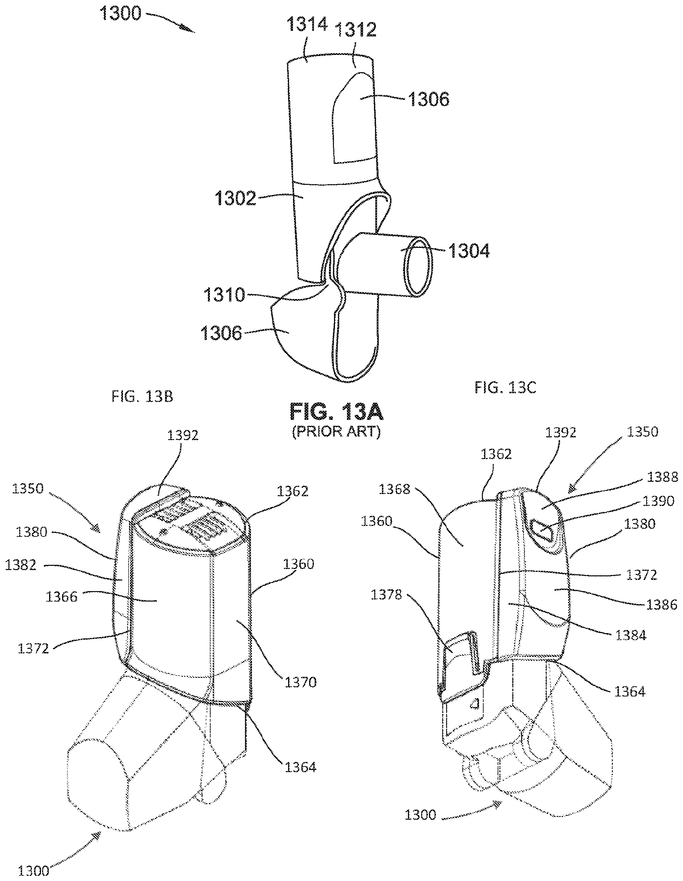

FIG. 13A is a perspective view of another type of known prior art inhaler 1300. In this example, the inhaler 1300 is a Teva Redihaler^{®}. The inhaler 1300 includes an actuator body 1302, a cylindrical mouthpiece 1304, a canister cover 1306 and a pivoting cap 1308 (shown in an open position). The pivoting cap is mounted on pivot points 1310 to rotate from an open position to a closed position to cover the mouthpiece 1304. The canister cover 1306 includes a top cover 1312 with vents 1314.

In this example, a user of the inhaler 1300 rotates the cap 1308 to the open position to uncover the mouthpiece 1304. The user then breathes out fully. The user then places the mouthpiece 1304 in their mouth and inhales deeply to release the medicament from the canister in the canister cover 1306. The inhaler 1300 has a membrane (not shown), that is opened by the inhalation and thus releases the medicament from the canister.

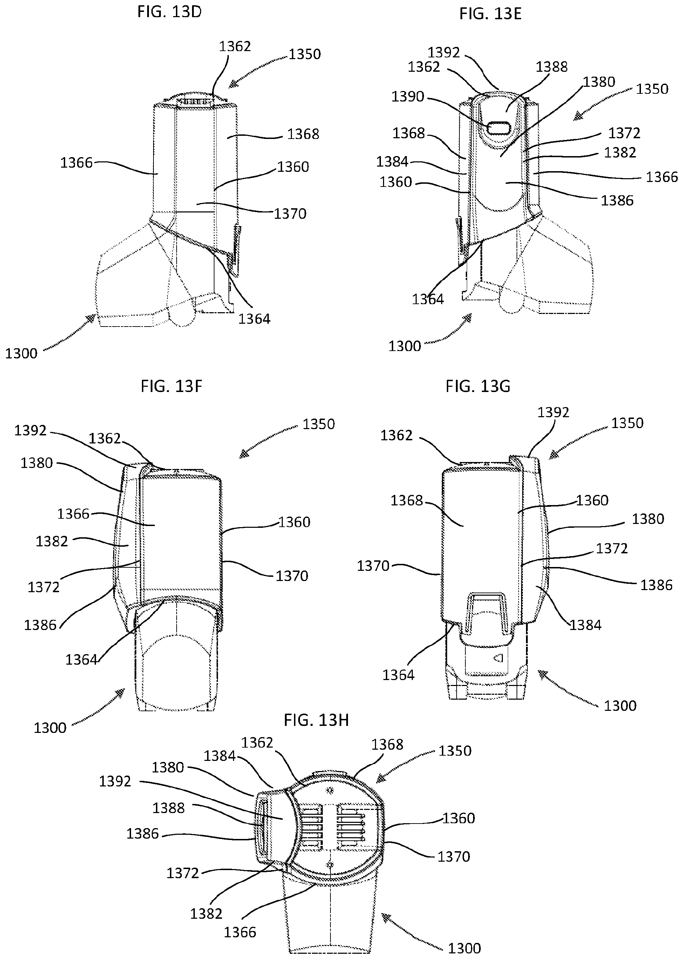

FIG. 13B is a front perspective view of another example type of modular adherence monitor 1350 attached to the inhaler 1300 shown in FIG. 13A, which is described below for clarifying only. FIG. 13C is a rear perspective view of the modular adherence monitor 1350 attached to the inhaler 1300. FIG. 13D is a side view of the modular adherence monitor 1350 attached to the inhaler 1300. FIG. 13E is an opposite side view of the modular adherence monitor 1350 attached to the inhaler 1300. FIG. 13F is a front view of the modular adherence monitor 1350 attached to the inhaler 1300. FIG. 13H is a rear view of the modular adherence monitor 1350 attached to the inhaler 1300. FIG. 13G is a top view of the modular adherence monitor 1350 attached to the inhaler 1300.

The adherence monitor 1350 includes a support body 1360 that has an open top end 1362 and an opposite open end 1364. The support body 1360 has a curved front wall 1366 and an opposite curved rear wall 1368. Two side walls 1370 and 1372 are joined to the front wall 1366 and rear wall 1368. The shape of the support body 1360 formed by the walls 1366, 1368, 1370, and 1372 is designed to fit over and around the canister cover 1306 of the inhaler 900 as shown in FIGs. 13B-13H.

The walls 1366, 1368, 1370 and 1372 wrap fully around the inhaler 1300 leaving the top cover 1312 and vents 1314 exposed. The rear wall 1368 includes a thumb push-tab 1378 at the bottom to assist in removing the adherence monitor 1350 from the inhaler 1300. When pressed, the monitor 1350 is pulled upwards relative to the inhaler 1300.

The side wall 1372 supports an electronics housing 1380. The electronics housing 1380 is formed from front and rear walls 1382 and 1384 and an adjoining side wall 1386. The side wall 1386 has an upper panel 1388 that includes an interface button 1390. Similar to the other example adherence monitors, the interface button 1390 may be pushed for operating different functions. A cover 1392 extends from the side wall 1372 to enclose the electronic components in the housing 1380.

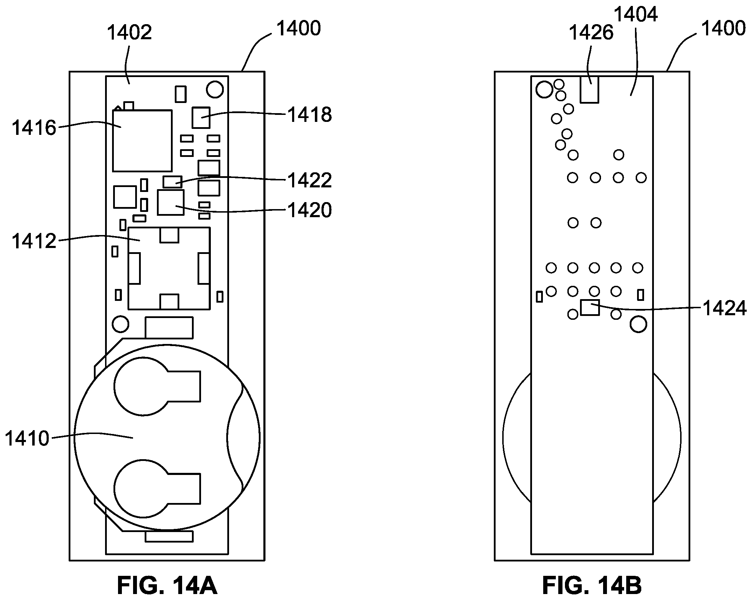

FIG. 14A shows a front surface 1402 of a circuit board 1400 mounted in the electronics housing 1380 in FIGs. 13B-13H. FIG. 14B shows a rear surface 1404 of the circuit board 1400. The front surface 1402 and rear surface 1404 of the circuit board 1400 have electronic components attached by soldering or other attachment mechanisms. The circuit board 1400 includes a battery 1410, a piezo-electrical bender circuit 1412, a communication module 1416, an accelerometer 1418, an auxiliary switch 1420, and an LED 1422 mounted on the front surface 1402. The circuit board 1400 includes an attachment detection IR sensor 1424 and a barometric pressure sensor 1426 mounted on the rear surface 1404.

The battery 1410 powers the electronic components on the circuit board 1400. In this example, the battery 1410 is a coin cell battery. The adherence monitor 1350 has an extra-low power inventory mode that is used to preserve the battery charge during the shelf life period. The piezo-electric bender circuit 1412 gives the user audible feedback from the use of the inhaler 1300 and the adherence monitor 1350. The communication module 1416 is similar to the communications module 316 in FIG. 3 and includes a controller that executes the algorithms to collect data and operate the adherence module 1350 in accordance with the flow diagram in FIG. 8. The controller also controls the transmission of data to a client device such as an external computing device.

In this example, the accelerometer 1418 is a low power 3-axis accelerometer running all the time after exiting an initial storage/inventory mode. The accelerometer 1018 is mainly used for power management and also detects shaking of the inhaler 1300 and the adherence module 1350 with sufficient motion for actuation detection.

In this example, the interface button 1390 in FIGs. 13B and 13E rests above the auxiliary switch 1420. The LED 1422 illuminates the interface button 1390 and may be turned on and off to indicate different states. The interface button 1390 has multiple functions including toggling reminder sounds on/off, waking the adherence monitor 1350 from the storage state, and generating manual heartbeat events.

In this example, the barometric pressure sensor 1426 captures information about the inhalation of a user as the user inhales the medicament through the mouthpiece 1304 similar to the pressure sensor 326 explained above. The controller in the communication module 1016 attaches information from the pressure sensor 1426 to the event record associated with an actuation event of the inhaler 1300. The additional information is similar to the pressure sensors in the other example inhalers explained above.

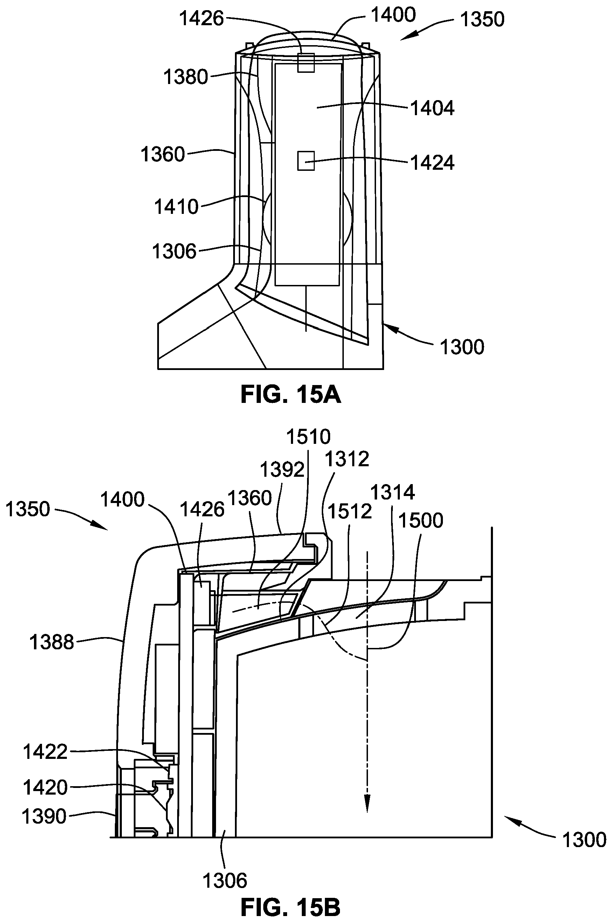

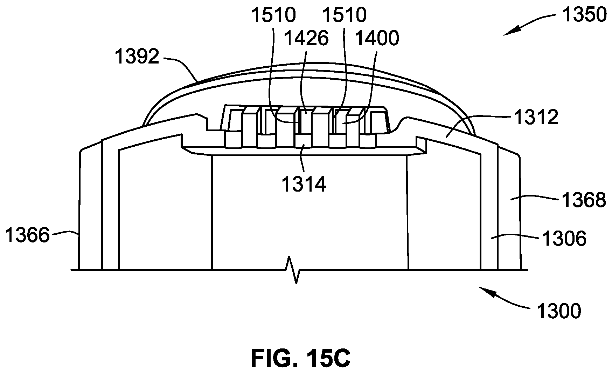

FIG. 15A is a cross section side view of the circuit board 1400 when the adherence monitor 1350 is inserted over the canister cover 1306. FIG. 13B is a close up cross section front view of the circuit board 1400 when the adherence monitor 1350 is inserted over the canister cover 1306. FIG. 15C is a close up side view of the pressure sensor 1426 in relation to the air vents 1314 of the inhaler 1300. As may be seen in FIG. 14B, the attachment detection IR sensor 1424 is placed on the rear surface 1404 of the circuit board 1400 between the circuit board 1400 and the canister cover 1306. The attachment detection IR sensor 1424 detects when the adherence monitor 1350 is inserted over the canister cover 1306 of the inhaler 1300.

An inhalation generates a large pressure drop within the inhaler 1300, which pulls air as shown by a dashed line 1500 through the vents 1314 at the top cover 1312 of the inhaler 1300. A series of ribs 1510 extend from the interior surface of the cover 1392 and are connected to the vents 1314 of the inhaler 1300. The ribs 1510 create a passage (shown by dashed line 1512) to connect the barometric pressure sensor 1426 to the vents 1314 in the inhaler to take advantage of the Venturi effect during an inhalation. The resulting pressure drop is registered by the barometric pressure sensor 1426 near the top of the circuit board 1400.

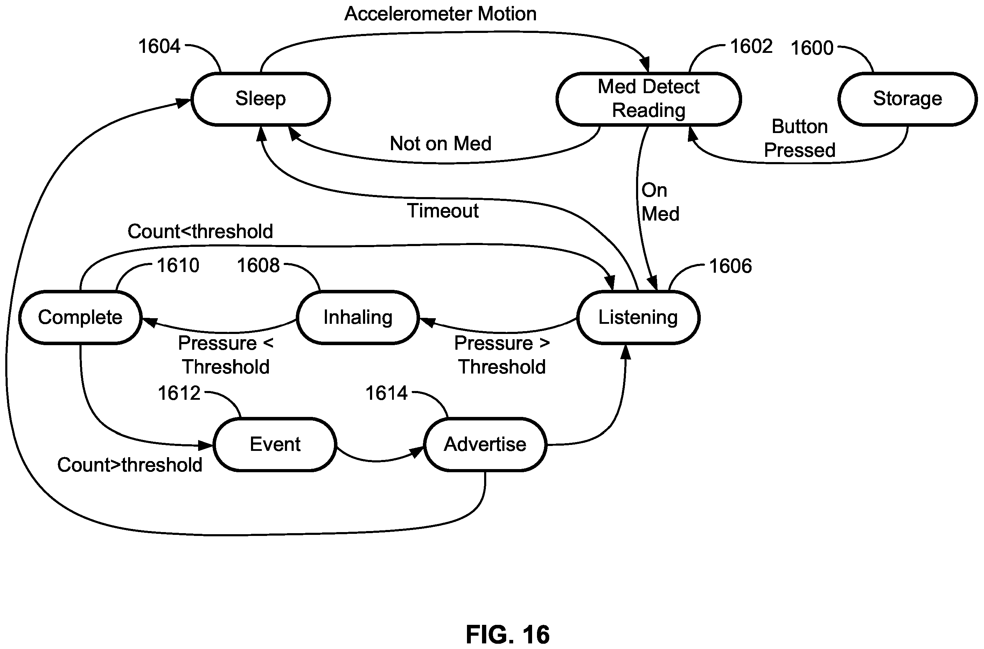

FIG. 16 is a state diagram the detection algorithm employed by the controller on the communications module 1416 in this example. The controller starts in a low-power storage mode 1600. As explained above, pressing the button 1390 thereby activating the limit switch 1320 exits the low-power storage mode 1600 to a monitor detection state 1602. The controller in the monitor detection state 1602 wakes the attachment detection IR sensor 1424. When awake, the IR sensor 1424 gates further sensing depending on the state. If the IR sensor 1424 does not detect a surface in proximity, indicating the monitor 1350 is not attached to the inhaler 1300, the routine proceeds to a sleep state 1604. Similar to the other example adherence monitors, the adherence monitor 1350 is primarily in the low-power sleep state 1604. The accelerometer 1418 is used to wake the adherence monitor 1350 from the sleep state 1604 and to detect shaking of the inhaler 1300. Pressing the button 1390 to activate the auxiliary switch 1420 also wakes the adherence monitor 1350.

If the IR sensor 1424 detects a surface in proximity to it, indicating that the adherence monitor 1350 is attached to the inhaler 1300, the routine proceeds to a listening state 1606. The barometric pressure sensor 1426 is activated and a timer started. In this example, the pressure sensor 1426 listens for a pressure drop indicating that an actuation and inhalation has begun. Thresholds are set within the firmware algorithm to filter out false signals. The barometric pressure sensor 1426 starts collecting data at a rate of 10Hz in this example, maintaining a moving average of the ambient pressure. When the pressure varies from the moving average by more than a configurable pressure activity threshold, an inhaling state 1608 is entered and the moving average is fixed at its current value. As long as the value from the pressure sensor 1426 stays at least a configurable hysteresis value underneath the pressure activity threshold value, the algorithm stays in the inhaling state 1608. After the pressure returns below the lower threshold, and if the number of inhalation samples is greater than a configurable duration threshold, then an inhalation complete state 1610 is entered. The inhalation event is recorded in the event state 1612. The event is advertised in an advertising state 1614. The monitor 1350 then returns to the listening state 1606. If, after a configurable timeout period after the actuation, no event has been detected, the algorithm stops measuring for an event and returns to the sleep state 1604.

Usage events contain a timestamp captured from the moment that the barometric pressure sensor 1426 detects actuation. If the accelerometer 1418 detects shaking prior to actuation, the shake intensity and shake duration are added to the usage event. If the barometric pressure sensor 1426 detects an inhalation around the time of actuation, either before, during, or after, the peak value of the pressure measurement, the duration of inhalation, and the time between the start of inhalation to the peak of inhalation are added to the usage event. All usage events will also include a battery measurement and temperature measurement captured from the controller at the time of actuation.

Although the example adherence monitors 100 in FIG. 1B, 950 in FIG. 9B, and 1350 in FIG. 13B are modular and may be attached or detached from their respective inhalers, it is to be understood that the components of the example adherence monitors may be integrated into inhalers such as the inhaler 10 in FIG. 1A, the inhaler 900 in FIG. 9A, or the inhaler 1300 in FIG. 13A. The principles described herein may be incorporated into other types of modular adherence monitors or integrated into other types of inhalers.

As explained above, each of the example adherence monitors collects data on inhaler actuation and provides time stamp and other related data. The collected data includes an inhaler actuation event with a unique identifier, a time stamp for the event, the ambient temperature, and sensor battery level. The controllers for each example adherence monitor also collect the number of wakeups and duration of time awake, which is useful for estimating battery life and determining if doses may not be sensing correctly. The controllers also collect some additional flags such as whether the attached inhaler was moved in the last 24 hours, whether the inhaler is attached to the medicament, and whether it is being held at the correct orientation as determined by the respective accelerometers.

Thus, the example data record may be in a format of "Event 1, 4:00 PM, July 25, 2019, 21C, 2.9V, 2 wakeups, 90 seconds awake, moved, on med, level." In addition, based on the knowledge of the sensor type, and its association by the user with the medicament via a user interface, the medicament may be known and added to the data record by either the external device or an external server that receives data from the adherence monitor. Additional data such as the number of doses, may also be appended to the data record by the external device or the external server. Finally, individual sensors collect additional data specific to the sensor. The example adherence monitors collect the shake duration and intensity of a shake of the medicament prior to use. As explained above, the barometric pressure sensors allow collecting the peak value of the inhalation and the duration of the inhalation. The example adherence monitors 950 and 1350 collect the time of peak as difference between inhalation start and peak value, as well as total volume of inhalation. The adherence monitor 100 captures the time between actuation (when the medicament is released) and inhalation start, as well as the number of seconds the medicament actuator is pressed. With this data, a health care provider may determine whether the patient is using their medicament as instructed with proper inhalation technique. By evaluating trends in this data, health care providers can provide instruction on more effective use of the medication, or may determine that changes to their treatment are necessary.

Another example of a metric of the data collected by the example adherence monitors is a first second volume. The first second volume is the volume in the first part of the inhalation, which is most relevant for the inhalation, and gives a more complete picture of the inhalation profile. First second volume refers to the volume inhaled during the initial samples of the inhalation (after the threshold is reached). Once the inhalation detection algorithm starts (either gated by the motion detection for the Symbicort inhaler 10 in FIG. 1A or the Redihaler inhaler 1300 in FIG. 13A, or by the limit switch for the Easyhaler inhaler 900 in FIG. 9A), the barometric pressure sensor first collects a baseline by capturing a few samples and averaging them together. This baseline is continuously updated with new readings and the moving average is maintained, as the barometric pressure sensor captures a new reading every 100ms in this example. When the value of the pressure reading drops by more than half of a pre-set threshold, then future readings do not contribute to the moving average. When the value of the pressure reading drops to the threshold, the inhalation is considered to begin.

The first second volume is the sum of pressure differences from the baseline for the first 10 samples (1 second) after the threshold is reached. As an example, if the baseline pressure moving average hovers around 98000 pascals, and the threshold is 16, then when the user starts to inhale, any pressure reading below 1/2 the threshold (97992) does not contribute to the moving average, and any pressure reading below 97984 counts as the beginning of the inhalation. This is done so that a slow inhalation will not adjust the moving average, which would make it difficult to hit the threshold. It also allows the system to adjust to natural environmental pressure changes, such as changes in elevation.

This metric can be used to determine a more accurate representation of the breath profile, as most inhalations start with a strong breath and taper off slowly after the peak is reached. The patient typically dispenses the medication from the inhaler in the first second of the inhalation as well, so this metric allows the collection of an additional piece of data related to the time during the inhalation that is most important. If the user dispenses the medication from the inhaler in the first second but has a slow breath throughout, or a slowly increasing one, then the first second volume can assist with diagnosing a condition in which the patient is not timing their breathing well.

The data collected by the adherence monitor may be live data, in which all of the sensor values are streamed over the Bluetooth connection to a remote device such as a smart phone. This enables use of the adherence monitor for collecting more data about the inhaler technique of the patient, and allows for training the patient on proper use of the inhaler.

Data sent in the live data feature may include the state of each button on the adherence monitor, the physical orientation of the inhaler in three dimensions, a Boolean value of whether the orientation is correct for the inhaler, a Boolean value of whether the inhaler is attached to the medication canister, a Boolean value of whether the inhaler is being shaken, a Boolean value of whether the sensor is currently detecting an inhalation, and a current pressure reading as well as a baseline of the pressure. Thus, for the example adherence monitor 100 in FIGs. 2-5, the orientation of the inhaler 10 may be determined by the accelerometer 318, whether the inhaler 10 is attached to the adherence monitor 100 may be determined by the attachment detection IR sensor 332, and the pressure and baseline pressure may be determined by the barometric pressure sensor 326.

This data is sent at regular intervals. The controller of the adherence monitor may be programmed to determine whether the current data is different from the data gathered in a previous interval. If the data is the same, the controller conserves battery power by not sending an update.

The live data may be used to evaluate whether proper technique has been followed. The technique in using the specific inhaler may be evaluated by the live data. For example, the data may indicate the adherence monitor is not attached to the inhaler. The live data may indicate whether the adherence monitor is in the correct orientation for providing a dose. The live data may indicate whether the user is inhaling, and if so at what intensity. The live data may also demonstrate that whether the user inhaled with appropriate metrics such as duration, intensity, and timing of actuation to have achieved a good deposition of the medication. Additional analysis could show whether the user was deviating in their typical inhalation profile, indicating an impending exacerbation, or improvement or worsening of their condition.

The live data may be used to determine whether a specific inhaler model was shaken first (or not shaken in the case of the Redihaler inhaler 1300 in FIG. 13A. For example, when the adherence monitor 1350 is woken through motion detection, the shake detection algorithm is turned on. The algorithm monitors the accelerometer 1418 at a regular interval and calculates the magnitude of acceleration. If the magnitude reaches a threshold value and maintains that threshold value for a minimum period, then a shake is in progress. The live data includes the accelerometer values, as well as a binary value of whether the adherence monitor 1350 is being shaken.

Another example is whether the Easyhaler inhaler 900 in FIG. 9A is being primed. The live data includes a binary value of the state of the limit switch 1022 on the Easyhaler inhaler adherence monitor 950. When the actuator on the inhaler 900 is being pressed to dispense the medication, this binary value changes status, indicating that priming is taking place.

Another example of a specific technique is determining whether a user is dispensing the medication for the Symbicort inhaler 10 in FIG. 1A. The actuation detection IR sensor 338 determines the state of the canister 14 of the inhaler 10 and whether it is depressed. This data is presented as a binary value in the live data, as well as the raw readings of the IR sensor 338. When the user presses the canister 14 to dispense medication, this value changes. By pairing this data with the inhalation data collected from the pressure sensor 326 and the timing of each reading, it is possible to determine if the user is priming the inhaler 10 without inhaling, or inhaling without dispensing medication, inhaling too weakly, or timing inhalation with medication dispensing properly to receive a complete dose.

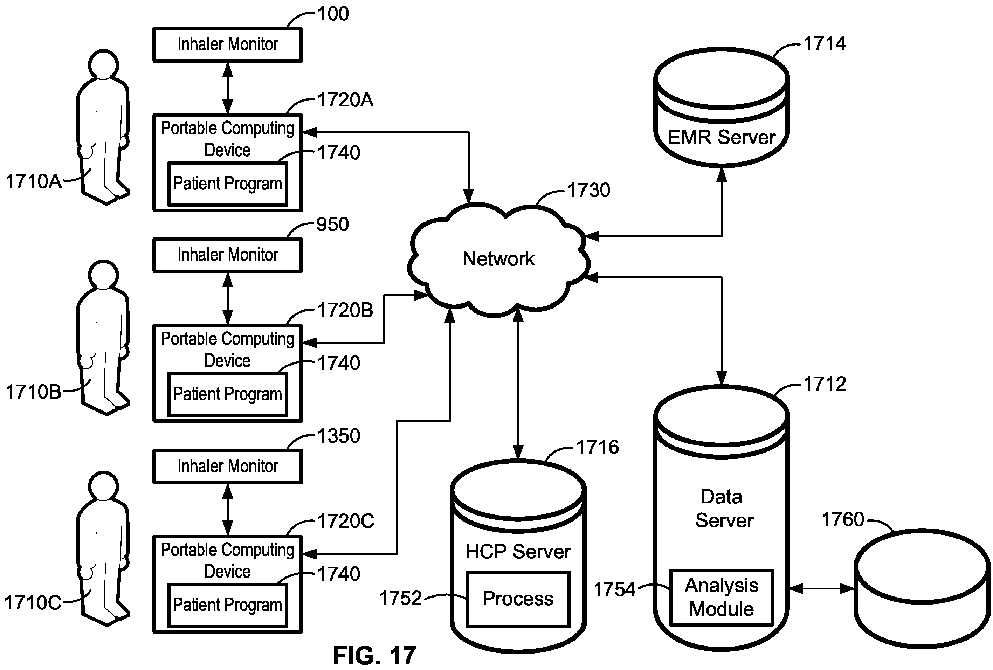

FIG. 17 is a block diagram of an example health care system 1700 for obtaining adherence data and other data from adherence monitors such as the adherence monitor 100 from patients using inhalers such as the inhaler 10 in FIG. 1A, the inhaler 900 in FIG. 9A, or the inhaler 1300 in FIG. 13A. The health care system 1700 includes multiple inhalers such as the inhaler 10 in FIG. 1A, an inhaler 900 in FIG. 9A, or the inhaler 1300 in FIG. 13A or any other types of inhalers that each may be actuated to provide drug dosages to a corresponding user or patient 1710a, 1710b, and 1710c. The health care system 1700 includes a data server 1712, an electronic medical records (EMR) server 1714, a health or home care provider (HCP) server 1716, and corresponding patient computing devices 1720a, 1720b, and 1720c. The patient computing device 1720a is in proximity to the inhaler 10 and attached adherence monitor 100 in this example. Similarly, the patient computing devices 1720b and 1720c are in proximity to the inhalers 900 and 1300, and attached adherence monitors 950 and 1350. In the system 1700, these entities are all connected to, and configured to communicate with each other over a wide area network 1730, such as the Internet. The connections to the wide area network 930 may be wired or wireless. The EMR server 1714, the HCP server 1716, and the data server 1712 may all be implemented on distinct computing devices at separate locations, or any subcombination of two or more of those entities may be co-implemented on the same computing device.

The patient computing devices 1720a, 1720b, and 1720c may be a personal computer, mobile phone, tablet computer, or other device. The patient computing device 1720a is configured to intermediate between the patient 1710a and the remotely located entities of the system 1700 over the wide area network 1730. In the implementation of FIG. 17, this intermediation is accomplished by a software application program 940 that runs on the patient computing device 1720. A patient program 1740 operated by the patient computing devices 1720a, 1720b, and 1720c may be a dedicated application referred to as a "patient app" or a web browser that interacts with a website provided by the health or home care provider. The system 1700 may include other inhalers and adherence monitors (not shown) associated with respective patients who also have respective associated computing devices and associated HCP servers (possibly shared with other patients). All the patients/inhaler users in the system 1700 may be managed by the data server 1712.

As explained above, the adherence data from the monitors 100, 950, and 1350 may be correlated with the application of drug doses from the inhalers 10, 900, and 1300. Additional data from the monitors 100, 950, and 1300 may be collected by the computing devices 1720a, 1720b, and 1720c to track the technique of the patient in applying the drug as explained above in relation to an analysis module 1754. Such data may be transmitted by the computing devices 1720a, 1720b, and 1720c to the data server 1712. The analysis module 1754 may provide analysis of the collected data from the routine in FIG. 8 such as determining proper technique in use of the inhaler by individual patients.

In this example, the monitors 100, 950, and 1350 are configured to transmit the data collected from inhaler actuation from applying a drug dose to the respective patient computing devices 1720a, 1720b, and 1720c via a wireless protocol, which receives the data as part of the patient program 1740. The patient computing devices 1720a, 1720b, and 1720c then transmit the data to the data server 1712 according to pull or push model. The data server 1712 may receive the data from the computing devices 1720a, 1720b, and 1720c according to a "pull" model whereby the computing devices 1720a, 1720b, and 1720c transmit the physiological data in response to a query from the data server 1712. Alternatively, the data server 1712 may receive the physiological data according to a "push" model whereby the computing devices 1720a, 1720b, and 1720c transmit the event data to the data server 1712 as soon as it is available after a dose is administered from the inhaler. Further, the data server 1712 may access databases 1760 to store collected and analyzed data relating to the patients 1710a, 1710b, and 1710c and big data relating to overall populations of patients.

Data received from the patient computing devices 1720a, 1720b, and 1720c is stored and indexed by the data server 1712 so as to be uniquely associated with the monitors 100, 950, and 1350 and therefore distinguishable from data collected from any other monitors in the system 1700. In this regard, although only three inhalers and monitors are illustrated in FIG. 17 for ease of explanation, the system 1700 may include many more inhalers and monitors. The data server 1712 may be configured to calculate summary data for each dose application from the data received from the monitor 100. The data server 1712 may also be configured to receive data from the patient computing devices 1720a, 1720b, and 1720c including data entered by the respective patients 1710a, 1710b, and 1710c, behavioral data about the patient, or dose/summary data.

The EMR server 1714 contains electronic medical records (EMRs), both specific to the patients 1710a-c and generic to a larger population of patients with similar disorders to the patients 1710a-c. An EMR, sometimes referred to as an electronic health record (EHR), typically contains a medical history of a patient including previous conditions, treatments, co morbidities, and current status. The EMR server 1714 may be located, for example, at a hospital where any of the patients 1710a-c has previously received treatment. The EMR server 17914 is configured to transmit EMR data to the data server 1712, possibly in response to a query received from the data server 1712.

In this example, the HCP server 1716 is associated with the health/home care provider (which may be an individual health care professional or an organization) that is responsible for the patient's respiratory therapy. An HCP may also be referred to as a DME or HME (domestic/home medical equipment provider). The HCP server 1716 may host a process 1752 that is described in more detail below. One function of the HCP server process 1752 is to transmit data relating to the patients 1710a-c to the data server 1712, possibly in response to a query received from the data server 1712.

In some implementations, the data server 1712 is configured to communicate with the HCP server 1716 to trigger notifications or action recommendations to an agent of the HCP such as a nurse, or to support reporting of various kinds. Details of actions carried out are stored by the data server 1712 as part of the engagement data. The HCP server 1716 hosts an HCP server process 1752 that communicates with the analysis module 1754 and the patient programs 940.

For example, the HCP server process 1752 may provide adherence analysis on whether an inhaler is correctly operated. Also, the HCP server process 1752 may include the ability to monitor the patient use of the inhaler in accordance with compliance rules that specify the required inhaler usage over a compliance period, such as 30 days, in terms of a minimum number of doses, such as four times, for some minimum number of days, e.g. 21, within the compliance period. The summary data post-processing may determine whether the most recent time period is a compliant session by comparing the usage time with the minimum duration from the compliance rule. The results of such post-processing are referred to as "compliance data." Such compliance data may be used by a health care provider to tailor therapy that may include the inhaler and other mechanisms. Other actors such as payors may use the compliance data to determine whether reimbursement may be made to a patient.

As may be appreciated data in the data server 1712, EMR server 1714 and HCP server 1716 is generally confidential data in relation to the patients 1710a-c. Typically, the patients 1710a-c must provide permission to send the confidential data to another party. Such permissions may be required to transfer data between the servers 1712, 1714 and 1716 if such servers are operated by different entities.

As used in this application, the terms "component," "module," "system," or the like, generally refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller, as well as the controller, can be a component. One or more components may reside within a process and/or thread of execution, and a component may be localized on one computer and/or distributed between two or more computers. Further, a "device" can come in the form of specially designed hardware; generalized hardware made specialized by the execution of software thereon that enables the hardware to perform specific function; software stored on a computer-readable medium; or a combination thereof.

The terminology used herein is for the purpose of describing particular embodiments only, and is not intended to be limiting of the invention. As used herein, the singular forms "a," "an," and "the" are intended to include the plural forms as well, unless the context clearly indicates otherwise. Furthermore, to the extent that the terms "including," "includes," "having," "has," "with," or variants thereof, are used in either the detailed description and/or the claims, such terms are intended to be inclusive in a manner similar to the term "comprising."

Unless otherwise defined, all terms (including technical and scientific terms) used herein have the same meaning as commonly understood by one of ordinary skill in the art. Furthermore, terms, such as those defined in commonly used dictionaries, should be interpreted as having a meaning that is consistent with their meaning in the context of the relevant art, and will not be interpreted in an idealized or overly formal sense unless expressly so defined herein.

While various embodiments of the present technology have been described above, it should be understood that they have been presented by way of example only. Although the technology has been illustrated and described with respect to one or more implementations, equivalent alterations and modifications will occur or be known to others skilled in the art upon the reading and understanding of this specification and the annexed drawings. In addition, while a particular feature of the technology may have been disclosed with respect to only one of several implementations, such feature may be combined with one or more other features of the other implementations as may be desired and advantageous for any given or particular application.

## Claims

1. An adherence monitor (100) for attachment to an inhaler (10) having a drug canister (14) covered by a canister cover, an actuator (12) holding the drug canister (14), the actuator (12) having a mouthpiece (20), and a dosing device operable to allow the drug canister (14) to be actuated to release a dose, the adherence monitor (100) comprising:
an inhaler attachment detection IR sensor (332) operable to detect when the inhaler (10) is attached to the adherence monitor (100);
an actuation detection IR sensor (338) operable to sense the physical movement of the drug canister (14) when actuated;
an inhalation data sensor (326) operable to sense air pressure change created by inhalation of the dose from the actuation;
a battery (310) coupled to the inhaler attachment detection IR sensor (332), the actuation detection IR sensor (338) and the inhalation data sensor (326);
a controller (316) coupled to the battery (310), to the inhaler attachment detection IR sensor (332), and to the actuation detection IR sensor (338) and the inhalation data sensor (326) to record an actuation event; and
an accelerometer (318) for power management of the adherence monitor (100) coupled to the controller (316) and the battery (310), the accelerometer outputting a signal indicative of movement of the inhaler (10) prior to actuation,
the controller (316) being operable to activate the sensors (326, 332, 338) when the movement of the inhaler (10) is detected, wherein, when the acceleration sensed by the accelerometer (318) exceeds a predetermined threshold, the adherence monitor (100) exits a sleep mode (704), which is a low power state, and powers up the IR sensors (332, 338),
wherein
the adherence monitor (100) is initially in a one-time inventory mode (700) which represents the lowest power state in which the accelerometer (318) is turned off; and
pressing a user operable activation button (150) wakes the adherence monitor (100) from the one-time inventory mode (700) and moves a routine, which is executed by the controller (316), to an inhaler attachment detection reading state (702), from which the routine moves to the sleep mode (704) if no signal from the inhaler attachment detection IR sensor (332) is received due to the adherence monitor (100) not being attached to the inhaler (10).

2. The adherence monitor (100) of claim 1, wherein the inhaler (10) includes a shield (32) attached to the canister cover, and wherein the actuation detection IR sensor (338) detects the movement of the shield (32) as indicating actuation of the inhaler (10).

3. The adherence monitor (100) of any one of claims 1-2, wherein the inhalation data sensor (326) is a pressure sensor, wherein the controller (316) is operable to determine a pressure curve during inhalation of the dose from the inhaler (10).

4. The adherence monitor (100) of any one of claims 1-3, wherein the controller (316) is operable to apply a time stamp to collected data indicating actuation of the inhaler (10).

5. The adherence monitor (100) of any one of claims 1-4, further comprising a transceiver coupled to the controller (316), wherein the controller (316) is operable to send the data based on the actuation event to an external client device in communication with the transceiver.

6. The adherence monitor (100) of claim 5, wherein the external client device is a mobile computing device associated with the user, wherein the external client device executes an application to analyze the collected data to determine adherence.

7. The adherence monitor (100) of any one of claims 1-6, further comprising a body fitting over the canister cover of the inhaler (10).

8. The adherence monitor (100) of any one of claims 1-7, wherein the inhalation data sensor (326) is positioned on a circuit board (300) to be exposed to a gap between the adherence monitor (100) and the canister cover of the inhaler (10).

9. The adherence monitor (100) of claim 1, wherein the dosing device further includes a front shield surface (32), the adherence monitor (100) further comprising:
a pair of curved side walls (110, 112) that conform to the sides of the actuator's (12) cylindrical body, each of the side walls (110, 112) having an open front edge and a closed back edge;
a side arm (122) attached to one of the side walls to overlap the cylindrical body of the actuator (12), wherein the mouthpiece (20) of the inhaler (10) is accessible and the front shield surface is exposed; and
an electronics housing (140) attached to the closed back edges of the side walls (110, 112).

## Patentansprüche

1. Adhärenzmonitor (100) zum Befestigen an einem Inhalator (10), der einen Arzneimittelbehälter (14), der von einer Behälterabdeckung bedeckt ist, einen Aktuator (12), der den Arzneimittelbehälter (14) hält, aufweist, wobei der Aktuator (12) ein Mundstück (20) und eine Dosiervorrichtung aufweist, die betätigbar ist, um zu ermöglichen, dass der Arzneimittelbehälter (14) betätigt wird, um eine Dosis freizusetzen, wobei der Adhärenzmonitor (100) Folgendes umfasst:
einen IR-Sensor zur Erfassung der Befestigung (332), der betreibbar ist, um zu erkennen, wann der Inhalator (10) am Adhärenzmonitor (100) befestigt ist;
einen IR-Sensor zur Erfassung der Betätigung (338), der betreibbar ist, um die physische Bewegung des Arzneimittelbehälters (14) zu erfassen, wenn er betätigt wird;
einen Einatmungsdaten-Sensor (326), der betreibbar ist, um eine Luftdruckänderung zu erfassen, die durch Einatmen der Dosis von der Betätigung generiert wird;
eine Batterie (310), die an den IR-Sensor zur Erfassung der Befestigung (332), den IR-Sensor zur Erfassung der Betätigung (338) und den Inhalationsdaten-Sensor (326) gekoppelt ist;
einen Controller (316), der an die Batterie (310), den IR-Sensor zur Erfassung der Befestigung (332)und den IR-Sensor zur Erfassung der Betätigung (338) und den Inhalationsdaten-Sensor (326) gekoppelt ist, um ein Betätigungsereignis aufzuzeichnen; und
einen Beschleunigungsmesser (318) zur Energieverwaltung des Adhärenzmonitors (100), der an den Controller (316) und die Batterie (310) gekoppelt ist, wobei der Beschleunigungsmesser ein Signal ausgibt, das eine Bewegung des Inhalators (10) vor der Betätigung anzeigt,
wobei der Controller (316) betreibbar ist, um die Sensoren (326, 332, 338) zu aktivieren, wenn die Bewegung des Inhalators (10) erfasst wird, wobei, wenn die durch den Beschleunigungsmesser (318) erfasste Beschleunigung einen vorbestimmten Schwellenwert überschreitet, der Adhärenzmonitor (100) einen Schlafmodus (704) verlässt, der ein niedriger Energiezustand ist, und die IR-Sensoren (332, 338) einschaltet,
wobei sich der Adhärenzmonitor (100) anfänglich in einem einmaligen Bestandsmodus (700) befindet, der den niedrigsten Energiezustand darstellt, in dem der Beschleunigungsmesser (318) ausgeschaltet ist; und
durch Drücken einer vom Benutzer bedienbaren Aktivierungstaste (150) der Adhärenzmonitor (100) aus dem einmaligen Bestandsmodus (700) geweckt wird und eine Routine, die von dem Controller (316) ausgeführt wird, in einen Lesezustand zur Erfassung der Inhalatorbefestigung (702) bewegt, von dem aus die Routine in den Schlafmodus (704) übergeht, wenn kein Signal vom IR-Sensor zur Erfassung der Befestigung (332) empfangen wird, da der Adhärenzmonitor (100) nicht am Inhalator (10) befestigt ist.

2. Adhärenzmonitor (100) nach Anspruch 1, wobei der Inhalator (10) eine Abschirmung (32) aufweist, die an der Behälterabdeckung befestigt ist, und wobei der IR-Sensor zur Erfassung der Betätigung (338) die Bewegung der Abschirmung (32) als Anzeige der Betätigung des Inhalators (10) erfasst.

3. Adhärenzmonitor (100) nach einem der Ansprüche 1 bis 2, wobei der Inhalationdaten-Sensor (326) ein Drucksensor ist, wobei der Controller (316) betreibbar ist, um eine Druckkurve während der Inhalation der Dosis aus dem Inhalator (10) zu bestimmen.

4. Adhärenzmonitor (100) nach einem der Ansprüche 1 bis 3, wobei der Controller (316) betreibbar ist, um einen Zeitstempel auf gesammelte Daten anzuwenden, die eine Betätigung des Inhalators (10) angeben.

5. Adhärenzmonitor (100) nach einem der Ansprüche 1 bis 4, ferner umfassend ein Sendeempfangsgerät, das an den Controller (316) gekoppelt ist, wobei der Controller (316) betreibbar ist, um die Daten basierend auf dem Betätigungsereignis an eine externe Clientvorrichtung in Kommunikation mit dem Sendeempfangsgerät zu senden.

6. Adhärenzmonitor (100) nach Anspruch 5, wobei die externe Clientvorrichtung eine mobile Computervorrichtung ist, die dem Benutzer zugeordnet ist, wobei die externe Clientvorrichtung eine Anwendung ausführt, um die gesammelten Daten zum Bestimmen der Adhärenz zu analysieren.

7. Adhärenzmonitor (100) nach einem der Ansprüche 1 bis 6, ferner umfassend einen Körper, der über den Behälter des Inhalators (10) passt.

8. Adhärenzmonitor (100) nach einem der Ansprüche 1-7, wobei der Inhalationsdaten-Sensor (326) auf einer Leiterplatte (300) positioniert ist, um einem Spalt zwischen dem Adhärenzmonitor (100) und der Behälterabdeckung des Inhalators (10) ausgesetzt zu sein.

9. Adhärenzmonitor (100) nach Anspruch 1, wobei die Dosiervorrichtung ferner eine vordere Abschirmfläche (32) enthält, wobei der Adhärenzmonitor (100) ferner Folgendes umfasst:
ein Paar gewölbter Seitenwände (110, 112), die zu den Seiten des zylindrischen Körpers des Aktuators (12) passen, wobei jede der Seitenwände (110, 112) eine offene Vorderkante und eine geschlossene Hinterkante aufweist;
einen Seitenarm (122), der an einer der Seitenwände befestigt ist, um den zylindrischen Körper des Aktuators (12) zu überlappen, wobei das Mundstück (20) des Inhalators (10) zugänglich ist und die vordere Abschirmfläche freiliegt; und
ein Elektronikgehäuse (140), das an den geschlossenen Hinterkanten der Seitenwände (110, 112) befestigt ist.

## Revendications

1. Moniteur d'adhérence (100) destiné à être fixé à un inhalateur (10) doté d'une cartouche de médicament (14) couverte par un couvercle de cartouche, d'un actionneur (12) retenant la cartouche de médicament (14), l'actionneur (12) étant doté d'un embout buccal (20), et d'un dispositif de dosage servant à actionner la cartouche de médicament (14) pour libérer une dose, le moniteur d'adhérence (100) comprenant :
un capteur IR de détection de fixation d'inhalateur (332) servant à détecter quand l'inhalateur (10) est fixé au moniteur d'adhérence (100) ;
un capteur IR de détection d'actionnement (338) servant à détecter un mouvement physique de la cartouche de médicament (14) lorsqu'elle est actionnée ;
un capteur de données d'inhalation (326) servant à détecter un changement de pression atmosphérique créé par l'inhalation de la dose résultant de l'actionnement ;
une batterie (310) couplée au capteur IR de détection de fixation d'inhalateur (332), au capteur IR de détection d'actionnement (338) et au capteur de données d'inhalation (326) ;
un contrôleur (316) couplé à la batterie (310), au capteur IR de détection de fixation d'inhalateur (332) ainsi qu'au capteur IR de détection d'actionnement (338) et au capteur de données d'inhalation (326) pour enregistrer un événement d'actionnement ; et
un accéléromètre (318) de gestion d'alimentation du moniteur d'adhérence (100) couplé au contrôleur (316) et à la batterie (310), l'accéléromètre délivrant un signal indicatif d'un mouvement de l'inhalateur (10) avant l'actionnement,
le contrôleur (316) servant à activer les capteurs (326, 332, 338) lorsque le mouvement de l'inhalateur (10) est détecté, dans lequel, lorsque l'accélération détectée par l'accéléromètre (318) dépasse un seuil prédéterminé, le moniteur d'adhérence (100) sort d'un mode de veille (704), à savoir un état de faible consommation, et met sous tension les capteurs IR (332, 338),
dans lequel le moniteur d'adhérence (100) est initialement dans un mode d'inventaire unique (700) qui représente l'état d'alimentation le plus bas dans lequel l'accéléromètre (318) est éteint ; et
une pression sur un bouton d'activation actionnable par l'utilisateur (150) réveille le moniteur d'adhérence (100) hors du mode d'inventaire unique (700) et déplace un sous-programme, exécuté par le contrôleur (316), vers un état de lecture de détection de fixation d'inhalateur (702), à partir duquel le sous-programme passe au mode de veille (704) si aucun signal n'est reçu du capteur IR de détection de fixation d'inhalateur (332) en raison du fait que le moniteur d'adhérence (100) n'est pas fixé à l'inhalateur (10).

2. Moniteur d'adhérence (100) selon la revendication 1, dans lequel l'inhalateur (10) comporte une protection (32) fixée au couvercle de cartouche, et dans lequel le capteur IR de détection d'actionnement (338) détecte le mouvement de la protection (32) indicatif d'un actionnement de l'inhalateur (10).

3. Moniteur d'adhérence (100) selon l'une quelconque des revendications 1 et 2, dans lequel le capteur de données d'inhalation (326) est un capteur de pression, dans lequel le contrôleur (316) sert à déterminer une courbe de pression pendant l'inhalation de la dose délivrée par l'inhalateur (10).

4. Moniteur d'adhérence (100) selon l'une quelconque des revendications 1 à 3, dans lequel le contrôleur (316) sert à appliquer un horodatage aux données collectées indiquant un actionnement de l'inhalateur (10).

5. Moniteur d'adhérence (100) selon l'une quelconque des revendications 1 à 4, comprenant en outre un émetteur-récepteur couplé au contrôleur (316), dans lequel le contrôleur (316) sert à envoyer les données basées sur l'événement d'actionnement à un dispositif client externe en communication avec l'émetteur-récepteur.

6. Moniteur d'adhérence (100) selon la revendication 5, dans lequel le dispositif client externe est un dispositif informatique mobile associé à l'utilisateur, dans lequel le dispositif client externe exécute une application pour analyser les données collectées afin de déterminer l'adhérence.

7. Moniteur d'adhérence (100) selon l'une quelconque des revendications 1 à 6, comprenant en outre un corps monté sur le couvercle de cartouche de l'inhalateur (10).

8. Moniteur d'adhérence (100) selon l'une quelconque des revendications 1 à 7, dans lequel le capteur de données d'inhalation (326) est positionné sur une carte de circuit imprimé (300) pour être exposé à un espace entre le moniteur d'adhérence (100) et le couvercle de cartouche de l'inhalateur (10).

9. Moniteur d'adhérence (100) selon la revendication 1, dans lequel le dispositif de dosage comporte en outre une surface de protection avant (32), le moniteur d'adhérence (100) comprenant en outre :
une paire de parois latérales incurvées (110, 112) qui épousent les côtés du corps cylindrique de l'actionneur (12), chacune des parois latérales (110, 112) présentant un bord avant ouvert et un bord arrière fermé ;
un bras latéral (122) fixé à l'une des parois latérales pour chevaucher le corps cylindrique de l'actionneur (12), dans lequel l'embout buccal (20) de l'inhalateur (10) est accessible et la surface de protection avant est exposée ; et
un boîtier électronique (140) fixé aux bords arrière fermés des parois latérales (110, 112).
